# EUROPÄISCHE PATENTSCHRIFT

(11) **EP 3 218 678 B1**
(45) Veröffentlichungstag und Bekanntmachung des Hinweises auf die Patenterteilung: **19.06.2019**
(21) Anmeldenummer: 15798313.1
(22) Anmeldetag: 09.11.2015
(51) Int. Cl.: G01G 17/06

(54) **DOSIERWERKZEUG**
DOSING DEVICE
DISPOSITIF DE DOSAGE

(30) Priorität: 10.11.2014 CH 17372014
(43) Veröffentlichungstag der Anmeldung: 20.09.2017
(73) Patentinhaber: Chemspeed Technologies AG, 4414 Füllinsdorf (CH)
(72) Erfinder: GUELLER, Rolf, CH-5027 Herznach (CH); SCHNEIDER, Michael, CH-5070 Frick (CH); THALER, Thomas, CH-4242 Laufen (CH); SCHINDLER, Markus, CH-6373 Ennetbürgen (CH)
(74) Vertreter: Bohest AG
(86) Internationale Anmeldenummer: PCT/CH2015/000165
(87) Internationale Veröffentlichungsnummer: WO 2016/074105

(56) Entgegenhaltungen:
- EP-A1- 1 930 702
- WO-A1-03/098170
- DE-A1-102004 035 061

## Beschreibung

Die vorliegende Erfindung betrifft eine Vorrichtung zur Dosierung einer Substanz gemäss dem Oberbegriff des unabhängigen Anspruchs 1. Die Erfindung betrifft auch ein Dosierverfahren unter Verwendung der Vorrichtung sowie ein Dosierwerkzeug für den Einsatz in der Vorrichtung.

Eine Substanz- bzw. Verbindungsbibliothek oder Bibliothek von Katalysatoren ist das Herzstück eines jeden chemischen Unternehmens, insbesondere der Pharmaindustrie. Viele Verbindungen aus oft jahrzehntelanger Synthesetätigkeit sind hier abgelegt und bilden eine Quelle für viele Screening-Aktivitäten. Die gelagerten Substanzen bzw. Verbindungen sind oft sehr wertvoll, schwierig zu synthetisieren, teuer zu kaufen oder zu extrahieren, und oftmals sind die Substanzen weltweit in nur ganz geringen Mengen überhaupt vorhanden.

Pharmazeutische Unternehmen halten in ihren Substanzbibliotheken oft eine Million und mehr Substanzen vorrätig und setzen zur Verwaltung eigene Substanz-Management-Gruppen ein, deren Aufgabe es ist, benötigte Substanzmuster in oft kleinsten Mengen bis hinunter in den Sub-Milligramm-Bereich aus der Bibliothek bereitzustellen, z.B. für spezielle biologische Tests. Hierbei muss die benötigte Substanzmenge aus dem Vorratsbehälter der Bibliothek entnommen und in ein Transportgefäss abgefüllt werden (sogenannte 1:1-Abfüllung). Je nach Konsistenz bzw. physikalischen Eigenschaften der abzufüllenden Substanz kann dies bisher nur manuell erfolgen, wozu insbesondere bei grösseren Unternehmen ein verhältnismässig grosser zeitlicher und/oder personeller Aufwand erforderlich ist. Die Automation ist unter anderem deshalb schwierig, weil die Substanzen oft in sehr unterschiedlicher physischer Form bzw. Konsistenz vorliegen. Ausserdem besteht bei dieser manuellen Vorgehensweise immer ein erhebliches Kontaminationsrisiko in dem Sinn, dass Spuren einer Substanz von einem vorhergehenden Dosiervorgang in das Aufnahmegefäss eines nachfolgenden Abfüllgangs gelangen können (cross contamination). Die oft erforderliche extrem hohe Dosiergenaugkeit resp. die meist sehr geringen vorhandenen und entsprechend noch kleineren abzugebenden Substanzmengen stellen eine weitere Schwierigkeit bei der praktischen Arbeit dar. Eine weitere praktische Schwierigkeit besteht darin, dass die abzufüllenden bzw. zu dosierenden Substanzen ganz unterschiedliche Konsistenzen aufweisen können, welche jeweils den Einsatz von spezifischen Abfüllwerkzeugen erfordern.

In der WO 03/098170 A1 ist eine rechnergesteuerte Dosiervorrichtung beschrieben, die für Substanzen praktisch jeglicher Konsistenz (pulverförmig, flüssig, ölig, pastös, harzig) geeignet ist. In einer Ausführungsform ist die Dosiervorrichtung mit einem Nadelabfüllkopf ausgestattet, der an einer elektronischen Waage befestigt ist, wobei der Nadelabfüllkopf zusammen mit der Waage an einem Roboterarm montiert ist und von diesem in drei Dimensionen bewegt werden kann. Der Nadelabfüllkopf besitzt einen Nadelträger, an dem eine Vielzahl von Dosiernadeln in Form von nach unten offenen Röhrchen gruppenweise unterschiedlichen Durchmessers von 0.1-5 mm gehaltert ist. Zur Substanzaufnahme werden die Dosiernadeln bzw. Röhrchen mittels des Roboterarms in die aufzunehmende, in einem Vorratsbehälter vorliegende Substanz definiert eingetaucht bzw. eingestochen, wobei eine je nach Durchmesser der Röhrchen unterschiedliche Substanzmenge in die unteren Enden der Röhrchen eindringt und dort hängen bleibt. Daraufhin wird der Nadelabfüllkopf angehoben und über die Öffnung des zu befüllenden Aufnahmebehälters geführt. Anschliessend werden im Nadelabfüllkopf vorgesehene Kolben selektiv von oben in die Röhrchen eingeführt und dadurch die in den Röhrchen befindliche Substanz aus den Röhrchen in den Aufnahmebehälter ausgestossen. Die Dosierung erfolgt dabei schrittweise, d.h. die Entleerung der Röhrchen beginnt mit den Röhrchen grösseren Durchmessers und setzt sich nach Röhrchen kleineren Durchmessers hin fort, bis im Rahmen dieser schrittweisen Annäherung die gewünschte Dosiermenge erreicht ist. Die jeweils bei einem Dosierschritt (der Entleerung eines Röhrchens) in den Aufnahmebehälter zudosierte Substanzmenge wird über die Waage erfasst, und die elektronische Steuerung veranlasst, sofern die gewünschte Dosiermenge noch nicht erreicht ist, die schrittweise Entleerung der weiteren Röhrchen. Zusätzlich kann eine weitere Waage grösserer Präzision vorgesehen sein, auf der der Aufnahmebehälter steht und mit welcher die exakte Erfassung der tatsächlichen Dosiermenge möglich ist.

Die aus der WO 03/098170 A1 bekannte Dosiervorrichtung löst zwar das Problem unterschiedlicher Substanzkonsistenzen, ist aber für die Befüllung von kleinen und kleinsten Substanzbehältern mit Aufnahmevolumina im Milligramm- oder Sub-Milligramm-Bereich nicht oder zumindest nur bedingt geeignet. Ein Grund dafür liegt darin, dass solche Substanzbehälter (sog. Vials) sehr klein sind und normalerweise dicht nebeneinander gepackt in Aufnahmegestellen (Racks) angeordnet sind und die Substanzen oft in so geringen Mengen vorhanden sind, dass oft nicht einmal mehr der Boden bedeckt ist bzw. die Substanz an den Behälterwänden klebt. Da die Öffnungen dieser Substanzbehälter viel zu klein sind, um alle Röhrchen des Nadelfüllkopfs gleichzeitig aufzunehmen, müsste der Nadelfüllkopf für jeden einzelnen Dosiervorgang bewegt werden, um das entsprechende Röhrchen genau über dem Substanzbehälter zu positionieren. Dabei würden sich aber zumindest einige der jeweils benachbarten Röhrchen über einem oder mehreren der anderen Substanzbehälter im Aufnahmegestell befinden und die Gefahr von Kontamination dieser Substanzbehälter ist sehr hoch. Ein ähnliches Problem ergibt sich daraus, dass auch die Vorratsbehälter, aus denen die abzufüllenden Substanzen entnommen werden, eine gewisse Mindestgrösse haben müssen, damit die Röhrchen des Nadelabfüllkopfs in diese eindringen können. In Substanzbibliotheken liegen aber oftmals viele Substanzen in so geringen Mengen vor, dass die Vorratsbehälter einfach zu klein sind für den Einsatz der bekannten Dosiervorrichtung.

Ein weiteres grosses Problem liegt in der inhärenten Kontaminationsgefahr an sich. Da nicht mit Sicherheit ausgeschlossen werden kann, dass Substanzspuren in oder an den Röhrchen des Nadelabfüllkopfs haften bleiben, müssen jeweils vor dem Dosieren einer anderen Substanz die Röhrchen ausgewechselt werden. Dies ist jedoch relativ arbeitsaufwändig. Alternativ könnte natürlich auch der Nadeldosierkopf oder dessen Nadelträger als Ganzes ausgewechselt werden. Dies wäre aber ebenfalls aufwändig und würde vor allem die Bereithaltung einer grossen Zahl von Nadelabfüllköpfen bzw. Nadelträgern erfordern, was aus wirtschaftlichen Gründen natürlich unerwünscht wäre.

Durch die vorliegende Erfindung soll nun eine Dosiervorrichtung zur Verfügung gestellt werden, die die beschriebenen Nachteile der bekannten Dosiervorrichtungen vermeidet. Konkreter soll eine kostengünstige Lösung für eine Dosiervorrichtung angegeben werden, die für Substanzen praktisch jeglicher Konsistenz geeignet ist, auch kleinste Substanzmengen bis hinunter in den Milligramm- und Submilligramm-Bereich mit ausreichender Präzision dosieren kann und mit welcher Kontaminationsprobleme ohne besonderen Aufwand zuverlässig vermieden werden. Weitere Aufgaben der Erfindung bestehen in der Bereitstellung eines Dosierverfahrens unter Verwendung der Dosiervorrichtung sowie in der Bereitstellung eines Dosierwerkzeugs für den Einsatz in der Dosiervorrichtung.

Diese der Erfindung zugrunde liegende Aufgaben werden durch die erfindungsgemässe, im unabhängigen Anspruch 1 definierte Vorrichtung zur Dosierung einer Substanz, durch das erfindungsgemässe, im unabhängigen Anspruch 17 definierte Dosierwerkzeug, und durch das im unabhängigen Anspruch 22 definierte Verfahren zum Dosieren einer Substanz gelöst. Besonders vorteilhafte Weiterbildungen und Ausgestaltungen der erfindungsgemässen Dosiervorrichtung, des erfindungsgemässen Dosierwerkzeugs und des erfindungsgemässen Dosierverfahrens ergeben sich aus den jeweils abhängigen Ansprüchen.

Hinsichtlich der Dosiervorrichtung besteht das Wesen der Erfindung in Folgendem: Eine Vorrichtung zur Dosierung einer Substanz umfasst einen Dosierkopf und ein am Dosierkopf lösbar befestigtes Dosierwerkzeug zur Aufnahme und Abgabe von Substanz. Das Dosierwerkzeug ist als ein im Wesentlichen zylindrisches Röhrchen mit einem darin im Wesentlichen dicht gleitend verstellbar angeordneten Stempel ausgebildet, wobei der Stempel länger ist als das Röhrchen und am oberen Ende des Röhrchens aus diesem herausragt. Der Dosierkopf ist mit einem öffen- und schliessbaren ersten Greifwerkzeug zur lösbaren Einspannung des Röhrchens und mit einem öffen- und schliessbaren zweiten Greifwerkzeug zur lösbaren Einspannung des Stempels versehen. Ferner ist der Dosierkopf mit einer Heb- und Senkeinrichtung versehen, mittels welcher das zweite Greifwerkzeug relativ zum ersten Greifwerkzeug und dadurch der Stempel im Röhrchen heb- und senkbar ist. Ganz besonders vorteilhafterweise bestehen das Röhrchen und der Stempel aus Glas.

Durch die Ausbildung des Dosierwerkzeugs als Röhrchen mit integriertem Stempel können einerseits Substanzen praktisch jeglicher Konsistenz dosiert werden und wird anderseits das Kontaminationsproblem völlig eliminiert, da das Dosierwerkzeug aufgrund seiner extrem einfachen Konstruktion so kostengünstig herstellbar ist, dass es nach jedem Gebrauch weggeworfen werden kann.

Gemäss einer ersten vorteilhaften Ausführungsform weist der Dosierkopf eine interne Steuerung für die Heb- und Senkeinrichtung sowie das erste und das zweite Greifwerkzeug sowie mit der internen Steuerung zusammenarbeitende Bedienungsorgane auf. Dadurch ist der Dosierkopf als autonomes Handgerät einsetzbar.

Vorteilhafterweise weist dabei die interne Steuerung eine Schnittstelle zur Kommunikation mit einer externen Steuerung und/oder zu einer externen Ladestromquelle auf.

Zweckmässigerweise weist die Vorrichtung eine Waage für den Dosierkopf auf und ist die Waage mit einer Halterung für den Dosierkopf versehen. Dadurch kann der Dosierkopf einfach auf der Waage abgestellt werden.

Vorteilhafterweise ist dabei die Halterung mit elektrischen Kontakten ausgestattet, welche zur Zusammenarbeit mit entsprechenden elektrischen Kontakten am Dosierkopf ausgebildet sind. Dadurch kann auf einfache Weise eine Kommunikationsverbindung mit der Waage hergestellt werden und vorzugsweise auch die Aufladung eines zur Stromversorgung im Dosierkopf vorgesehenen Akkumulators erfolgen.

Gemäss einer zweiten vorteilhaften Ausführungsform weist die Vorrichtung eine Waage auf und ist der Dosierkopf so an der Waage angeordnet, dass die Waage das Gewicht des Dosierkopfs (mit bzw. ohne aufgenommene Substanz) misst.

Gemäss einer vorteilhaften Weiterbildung ist der Dosierkopf mit einer zweiten Heb-und Senkeinrichtung ausgestattet, mittels welcher das erste Greifwerkzeug zusammen mit der erstgenannten Heb- und Senkeinrichtung für das zweite Greifwerkzeug heb-und senkbar ist. Durch diese zweite Heb- und Senkvorrichtung ist eine feinfühligere Bewegung des Dosierwerkzeugs möglich als durch Bewegung des gesamten Dosierkopfs.

Zweckmässigerweise ist die Waage an einem Roboterarm befestigt, wobei der Dosierkopf mittels des Roboterarms verstellbar ist, und zwar vorzugsweise in allen drei Raumrichtungen.

Zweckmässigerweise ist die Vorrichtung mit einer Steuerelektronik für die Waage und die Heb- und Senkeinrichtung sowie das erste und das zweite Greifwerkzeug ausgestattet. Durch die Steuerelektronik kann die Dosiervorrichtung automatisch betrieben werden.

Vorteilhafterweise ist die Dosiervorrichtung zum Wägen eines Substanzaufnahmebehälters, der die abgegebene Substanz aufnimmt, mit einer weiteren Waage versehen. Mit dieser weiteren Waage, die vorzugsweise eine höhere Präzision aufweist als die erste Waage, kann die tatsächlich dosierte Substanzmenge kontrolliert und exakt gemessen werden.

Zweckmässigerweise weist das (Glas-)Röhrchen einen Innendurchmesser im Bereich von 0.1 bis 5 mm, vorzugsweise 0.1 bis 2 mm, noch bevorzugter 0.1 bis 1 mm, auf.

Weiter ist es vorteilhaft, wenn der Stempel als Glasstab oder als zumindest einseitig geschlossenes Glasrohr ausgebildet ist.

Gemäss einer besonders vorteilhaften Ausgestaltung weist das (Glas-)Röhrchen eine Wandstärke im Bereich von 0.03 bis 0.2 mm, vorzugsweise 0.03 bis 0.1 mm, auf. Zusätzlich oder alternativ weist das (Glas-)Röhrchen ein scharfkantig oder schneidenartig ausgebildetes Ende auf.

Gemäss einer weiteren vorteilhaften Ausgestaltung weist die Vorrichtung Drehmittel auf, welche das Dosierwerkzeug während seiner Anhebung und Absenkung durch die zweite Heb- und Senkeinrichtung um seine Längsachse drehen.

Gemäss einer weiteren vorteilhaften Ausgestaltung weist die Vorrichtung ein Aufnahmegestell für eine Anzahl von zumindest teilweise unterschiedlichen bzw. unterschiedlich dimensionierten Dosierwerkzeugen auf.

Hinsichtlich des Dosierwerkzeugs besteht das Wesen der Erfindung in Folgendem: Ein Dosierwerkzeug für den Einsatz in einer Dosiervorrichtung ist als ein im Wesentlichen zylindrisches Röhrchen mit einem darin im Wesentlichen dicht gleitend verstellbar angeordneten Stempel ausgebildet, wobei der Stempel länger ist als das Röhrchen und am einen Ende des Röhrchens aus diesem herausragt und vorzugsweise das Röhrchen nicht vollständig ausfüllt, so dass am anderen Ende des Röhrchens eine Dosierkammer frei bleibt.

Das besonders vorteilhafterweise ganz aus Glas und aus nur zwei Teilen bestehende Dosierwerkzeug ist aufgrund seiner konstruktiven Einfachheit sehr kostengünstig herstellbar und kann dadurch nach jedem Dosiervorgang weggeworfen werden, so dass Kontaminationsprobleme praktisch vollständig vermieden werden.

Gemäss einer vorteilhaften Ausgestaltung ist das (Glas-)Röhrchen zylindrisch ausgebildet und weist einen Innendurchmesser im Bereich von 0.1 bis 5 mm, vorzugsweise 0.1 bis 2 mm, noch bevorzugter 0.1 bis 1 mm, auf. Mit diesen Dimensionen lassen sich Dosiermengen von einigen Hundert mg bis hinunter in den Sub-Milligramm-Bereich realisieren.

Vorzugsweise ist der Stempel als Glasstab oder als zumindest einseitig geschlossenes Glasrohr ausgebildet. Dies führt zu einer einfachen Herstellbarkeit des Stempels.

Vorteilhafterweise weist das (Glas-)Röhrchen eine Wandstärke im Bereich von 0.03 bis 0.2 mm, vorzugsweise 0.03 bis 0.1 mm, auf. Durch diese verhältnismässig geringen Wandstärken wirkt der freie Rand des (Glas-)Röhrchens quasi als Schneide, was das Eintauchen bzw. besser Einstechen in Substanzen mit festerer Konsistenz sowie das Abschaben bzw. Abkratzen von Substanz von einer Behälterwand begünstigt. Zusätzlich oder alternativ weist das (Glas-)Röhrchen ein scharfkantig oder schneidenartig ausgebildetes Ende auf.

Hinsichtlich des Dosierverfahrens besteht das Wesen der Erfindung in Folgendem: Ein Verfahren zum Dosieren einer Substanz unter Verwendung der erfindungsgemässen Vorrichtung umfasst die folgenden Schritte:
- Einspannen eines Dosierwerkzeugs in den Dosierkopf durch Öffnen und Schliessen der Greifwerkzeuge
- Positionieren des Dosierwerkzeugs über einem Substanzvorratsbehälter
- Absenken des Dosierwerkzeugs zum Eintauchen bzw. Einstechen des Dosierwerkzeugs in eine im Substanzvorratsbehälter befindliche Substanz und dadurch Aufnehmen von Substanz in eine Substanzkammer des Dosierwerkzeugs, vorzugsweise unter der Kontrolle einer Waage
- Anheben des Dosierwerkzeugs
- gegebenenfalls Absenken des Stempels des Dosierwerkzeugs relativ zum Röhrchen zur Ausstossung überschüssiger Substanz, vorzugsweise unter der Kontrolle einer Waage
- Positionieren des Dosierwerkzeugs über einem Substanzaufnahmebehälter, und
- Absenken des Stempels des Dosierwerkzeugs relativ zum Röhrchen zur vollständigen Ausstossung von Substanz aus dem Dosierwerkzeug in den Substanzaufnahmebehälter.

Am Ende des Dosierens kann das Dosierwerkzeug durch Öffnen der Greifwerkzeuge aus dem Dosierkopf ausgeworfen werden.

Vorteilhafterweise wird die Dosierung in einzelnen Teildosierungen durchgeführt, wobei, vorzugsweise unter der Kontrolle einer Waage, eine schrittweise Annäherung an eine geforderte Soll-Dosiermenge erfolgt.

Zweckmässigerweise wird die vom Dosierwerkzeug aufgenommene Substanzmenge unter der Kontrolle einer Waage getrimmt.

Vorteilhafterweise wird die tatsächliche Menge der in den Substanzaufnahmebehälter dosierten Substanz mittels einer (weiteren) Waage gemessen.

Ganz besonders vorteilhafterweise wird das Dosierwerkzeug während des Eintauchens bzw. Einstechens in eine im Substanzvorratsbehälter befindliche Substanz und vorzugsweise auch während des Herausziehens aus der Substanz um seine Längsachse gedreht. Die Drehbewegung erleichtert das Einstechen im Falle von Substanzen mit relativ fester Konsistenz. Ausserdem verhindert sie ein Festsetzen des Dosierwerkzeugs in der Substanz und erlaubt bei gewissen Substanzen ein Herausbohren eines Substanzpfropfens.

Vorteilhafterweise wird vorzugsweise in einem Aufnahmegestell eine Anzahl von zumindest teilweise unterschiedlichen bzw. unterschiedlich dimensionierten Dosierwerkzeugen bereitgestellt und das einzuspannende Dosierwerkzeug aus diesen bereitgestellten Dosierwerkzeugen ausgewählt. Auf diese Weise können durch entsprechende Auswahl der Dosierwerkzeuge verschiedene Dosiermengen von einigen Hundert mg bis weit hinunter in den Sub-Milligramm-Bereich erreicht werden.

Im Folgenden wird die Erfindung anhand von in der Zeichnung dargestellten Ausführungsbeispielen detaillierter beschrieben. Es zeigen:
- Fig. 1 -: eine schematische Darstellung eines ersten Ausführungsbeispiels einer erfindungsgemässen Dosiervorrichtung,
- Fig. 2 -: einen schematischen Horizontalschnitt durch ein Greifwerkzeug der Dosiervorrichtung,
- Fig. 3 - 8 -: je eine schematische Darstellung der Dosiervorrichtung gemäss Fig. 1 in verschiedenen Phasen eines Dosiervorgangs,
- Fig. 9a-d -: je ein Dosierwerkzeug der Dosiervorrichtung gemäss Fig. 1 in verschiedenen Phasen eines Dosiervorgangs,
- Fig. 10a-d-: je ein Dosierwerkzeug der Dosiervorrichtung gemäss Fig. 1 in verschiedenen Phasen der Dosierung von Flüssigkeiten,
- Fig. 11a-b-: zwei Varianten des Dosierkopfs mit Dosierwerkzeug eines zweiten Ausführungsbeispiels einer erfindungsgemässen Dosiervorrichtung,
- Fig. 12a-c-: je eine schematische Schnittdarstellung des Dosierkopfs mit Dosierwerkzeug gemäss Fig. 11a in verschiedenen Phasen eines Dosiervorgangs,
- Fig. 13a-i-: je eine schematische Darstellung der Dosiervorrichtung mit einem Dosierkopf mit Dosierwerkzeug gemäss Fig. 11a in verschiedenen Phasen eines Dosiervorgangs,
- Fig. 14a-e -: einen Dosierkopf mit Dosierwerkzeug eines dritten Ausführungsbeispiels einer erfindungsgemässen Dosiervorrichtung in verschiedenen Phasen eines Dosiervorgangs und
- Fig. 15a-b -: zwei schematischen Skizzen zur Erläuterung der Aufnahme kristallisierter Substanzen und
- Fig. 16 -: einen Axialschnitt durch ein modifiziertes Röhrchen des Dosierwerkzeugs der Dosiervorrichtung.

Für die nachstehende Beschreibung gilt die folgende Festlegung: Sind in einer Figur zum Zweck zeichnerischer Eindeutigkeit Bezugszeichen angegeben, aber im unmittelbar zugehörigen Beschreibungsteil nicht erwähnt, so wird auf deren Erläuterung in vorangehenden oder nachfolgenden Beschreibungsteilen verwiesen. Umgekehrt sind zur Vermeidung zeichnerischer Überladung für das unmittelbare Verständnis weniger relevante Bezugszeichen nicht in allen Figuren eingetragen. Hierzu wird auf die jeweils übrigen Figuren verwiesen.

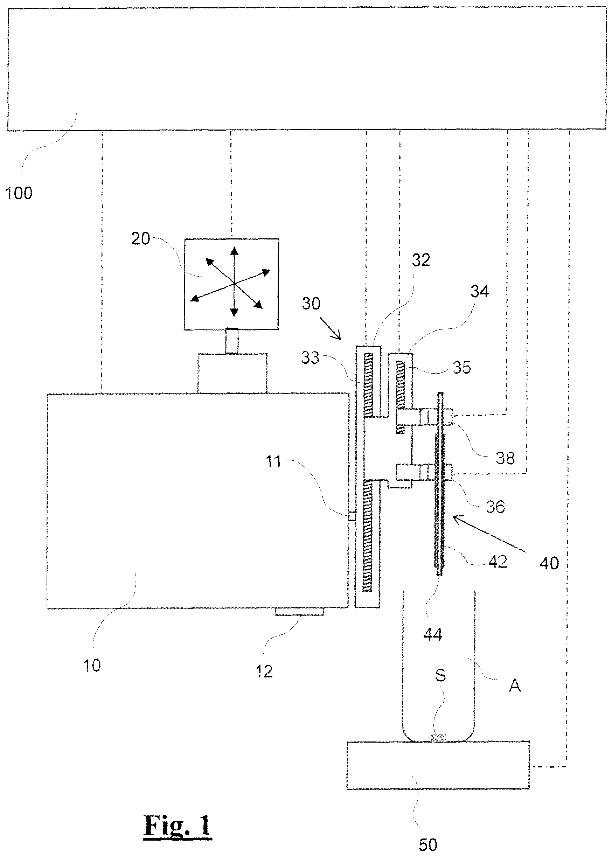

Die in Figur 1 dargestellte erste Ausführungsform der Dosiervorrichtung umfasst eine elektronische Waage 10, die an einem nur symbolisch durch einen Kasten angedeuteten Roboterarm 20 lösbar montiert ist. Zur Steuerung des Roboterarms 20 ist eine Steuerelektronik 100 vorgesehen. Mittels des Roboterarms 20 kann die Waage 10 in alle drei Raumrichtungen innerhalb der Reichweite des Roboterarms bewegt werden.

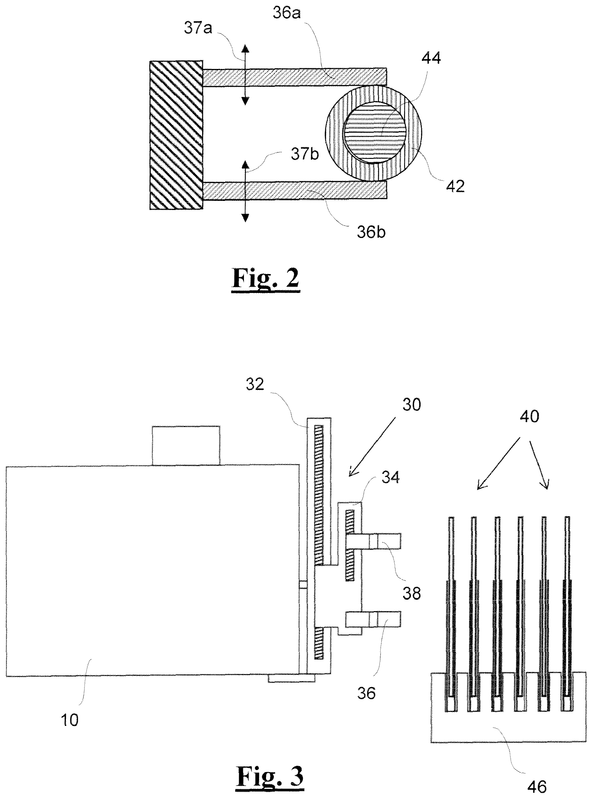

An der Waage 10 ist an einem mit der Wägezelle im Inneren der Waage verbundenen Waagarm 11 ein als Ganzes mit 30 bezeichneter Dosierkopf so montiert, dass die Waage das Gewichts des Dosierkopfs 30 und aller an diesem befestigten bzw. von diesem getragenen Komponenten misst. An der Waage 10 ist zu ihrer Schonung ein verstellbares Verriegelungsorgan 12 vorgesehen, das den Dosierkopf 30 relativ zur Waage 10 festlegt, wenn gerade keine Wägung erforderlich ist, siehe beispielsweise Fig. 3. Die Waage kann auch bei der Bewegung und/oder Aufstellung verriegelt werden.

Soweit und in dieser Allgemeinheit unterscheidet sich die erfindungsgemässe Dosiervorrichtung nicht von der bekannten Dosiervorrichtung gemäss der schon erwähnten WO 03/098170 A1, so dass der Fachmann hierzu keiner näheren Erläuterung bedarf.

Der Dosierkopf 30 umfasst im Wesentlichen eine erste Heb- und Senkeinrichtung 32 und eine zweite Heb- und Senkeinrichtung 34, welche an der ersten Heb- und Senkeinrichtung 32 montiert ist und mittels dieser (in Einsatzlage der Dosiervorrichtung vertikal) gehoben und gesenkt werden kann. An der zweiten Heb-und Senkeinrichtung 34 ist ein erstes Greifwerkzeug 36 ortsfest montiert. Ein zweites Greifwerkzeug 38 ist so an der zweiten Heb- und Senkeinrichtung 34 angeordnet, dass es mittels dieser (in Einsatzlage der Dosiervorrichtung vertikal) relativ zum ersten Greifwerkzeug 36 gehoben und gesenkt werden kann. Zur Hebe- bzw. Senkbewegung der beiden Greifwerkzeuge 36 und 38 sind die beiden Heb- und Senkeinrichtungen 32 und 34 mit durch nicht dargestellte Motoren drehbar angetriebenen Gewindespindeln 33 und 35 ausgestattet. Selbstverständlich können die Heb- und Senkeinrichtungen 32 und 34 auch anders realisiert sein.

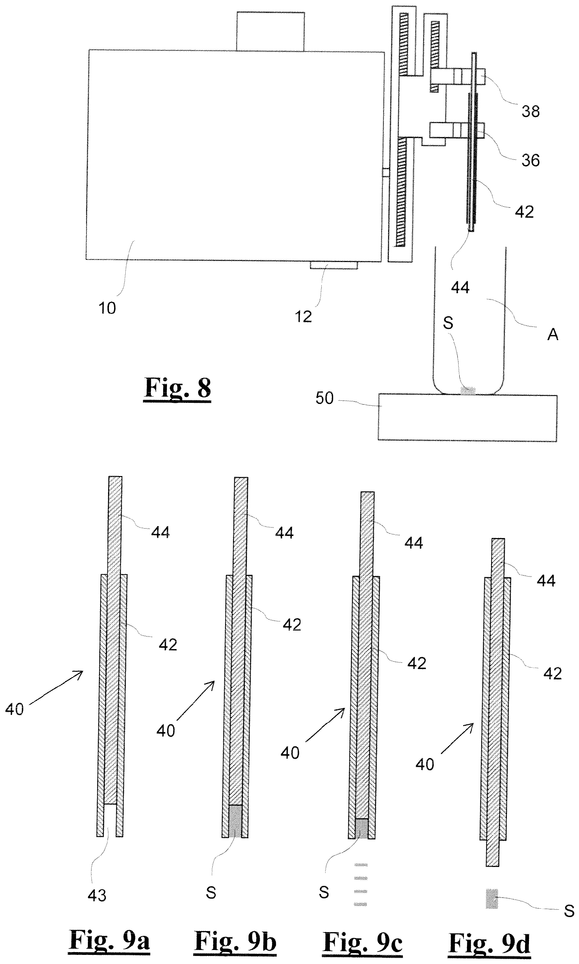

Für die eigentliche Substanzdosierung, also die Substanzaufnahme von einem Vorratsbehälter und die Substanzabgabe in einen Substanzaufnahmebehälter, ist ein speziell ausgebildetes Dosierwerkzeug 40 vorgesehen. Wie in Fig. 9a vergrössert dargestellt ist, besteht dieses Dosierwerkzeug 40 aus zwei Teilen, und zwar einem vorzugsweise im Wesentlichen zylindrischen Glasröhrchen 42 und einem ebenfalls aus Glas bestehenden Stempel 44. Der Glasstempel 44 ist etwas länger als das Glasröhrchen 42 und ragt aus dem oberen Ende des Glasröhrchens 42 heraus. Umgekehrt erstreckt sich der Glasstempel 44 (im Ausgangszustand) nicht über die gesamte Länge des Glasröhrchens 42, sondern lässt eine Dosierkammer 43 des Glasröhrchens 42 offen (Fig. 9a). Alternativ kann die Dosierkammer 43 auch erst während des Dosiervorgangs durch (begrenztes) Herausziehen des Glasstempels 44 aus dem Glasröhrchen 42 gebildet werden, wobei die Grösse bzw. das Volumen der Dosierkammer entsprechend den jeweiligen Erfordernissen eingestellt werden kann.

Der Querschnitt des Glasstempels 44 ist an den Innenquerschnitt des Glasröhrchens 42 angepasst, so dass der Glasstempel 44 als Kolben wirkt. Der Glasstempel 44 kann als voller Glasstab oder als zumindest an seinem innerhalb des Glasröhrchens 42 liegenden Ende geschlossenes Glasrohr ausgebildet sein. Ferner kann der Glasstempel prinzipiell auch in Form einer gläsernen Kolbenscheibe und einer gläsernen Kolbenstange ausgebildet sein.

Das Dosierwerkzeug 40 kann grundsätzlich auch aus einem anderen Material als Glas, z.B. aus Kunststoff, gebildet sein. Glas ist jedoch praktisch in allen Fällen chemisch inert und weist bei geeigneten Abmessungen auch eine gewisse Elastizität auf Im Folgenden wird die Erfindung deshalb durchgehend anhand eines aus Glas bestehenden Dosierwerkzeugs beschrieben.

Im Einsatz der erfindungsgemässen Dosiervorrichtung wird das Dosierwerkzeug 40 an seinem Glasröhrchen 42 vom ersten Greifwerkzeug 36 festgehalten. Das zweite Greifwerkzeug 38 hält den Glasstempel 44 fest.

Wie der Fig. 2 zu entnehmen ist, umfasst das erste Greifwerkzeug 36 im Wesentlichen zwei Klemmbacken 36a und 36b, die zwischen sich das Glasröhrchen 42 des Dosierwerkzeugs 40 festklemmen. Die beiden Klemmbacken 36a und 36b können mittels eines hier nur durch Pfeile 37a und 37b angedeuteten Antriebs voneinander weg bzw. aufeinander zu bewegt werden.

Das zweite Greifwerkzeug 38 ist analog dem ersten Greifwerkzeug 36 ausgebildet und daher nicht separat dargestellt.

Die Fig. 1 zeigt ferner noch einen Substanzaufnahmebehälter A, der auf einer zweiten elektronischen Waage 50 steht. Bei der zweiten Waage 50 handelt es sich vorteilhafterweise um eine Analysenwaage, deren Auflösung und Genauigkeit mindestens so hoch, vorzugsweise höher ist als die Auflösung und Präzision der ersten Waage 10. Vorteilhafterweise ist die Genauigkeit der zweiten Waage 50 etwa 0.01 mg, vorzugsweise sogar etwa 0.001 mg.

Die erste Waage 10, die beiden Heb- und Senkeinrichtungen 32 und 34, die beiden Greifwerkzeuge 36 und 38 und die zweite Waage 50 werden von der Steuerelektronik 100 funktionell gesteuert. Die praktische Implementierung der erforderlichen Funktionen (Auslesen der Waagen, Bewegen des Roboterarms, Bewegen der Heb-und Senkeinrichtungen, Schliessen und Lösen der Greifwerkzeuge) sind im normalen Könnensbereich des Steuerungsfachmanns und bedürfen daher keiner näheren Erläuterung.

Im Folgenden wird ein unter Einsatz der erfindungsgemässen Dosiervorrichtung durchgeführtes Dosierverfahren anhand der Figuren 3-9d näher beschrieben.

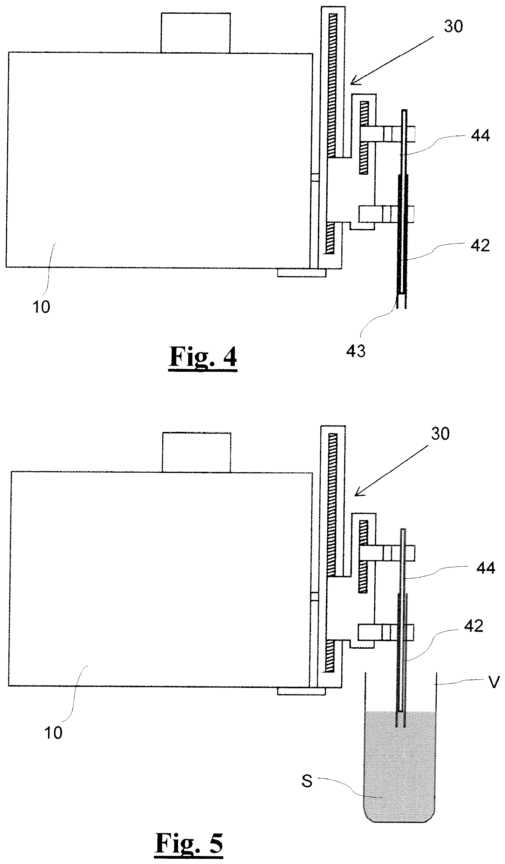

In dem in Fig. 3 dargestellten Ausgangszustand befindet sich noch kein Dosierwerkzeug 40 am Dosierkopf. Eine Anzahl von Dosierwerkzeugen 40 ist in einem Aufnahmegestell (Rack) 46 vorrätig gehalten. Mittels des Roboterarms 20 wird der Dosierkopf 30 an das Aufnahmegestell 46 herangefahren und die beiden Greifwerkzeuge 36 und 38 erfassen ein Dosierwerkzeug 40 und halten es fest. Das Dosierwerkzeug 40 wird dann durch Verfahren des Roboterarms 20 aus dem Aufnahmegestell 46 entnommen (Fig. 4). Selbstverständlich könnte umgekehrt auch das Aufnahmegestell 46 mittels einer anderen Transportvorrichtung an den Dosierkopf 30 herangefahren und dann wieder weggefahren werden.

Anschliessend wird das Dosierwerkzeug 40 mittels des Roboterarms 20 oberhalb eines bereitgestellten, die zu dosierende Substanz enthaltenden Vorratsbehälters V positioniert und dann soweit abgesenkt, bis das Glasröhrchen 42 des Dosierwerkzeugs 40 in die Substanz S eintaucht bzw., je nach Konsistenz der Substanz, einsticht (Fig. 5). Dabei füllt sich die Dosierkammer 43 des Glasröhrchens 42 mit Substanz S (Fig. 9b). Gegebenenfalls wird zuvor zur Bildung der Dosierkammer 43 mittels der zweiten Heb- und Senkeinrichtung 34 der Glasstempel 44 etwas aus dem Glasröhrchen 42 herausgezogen.

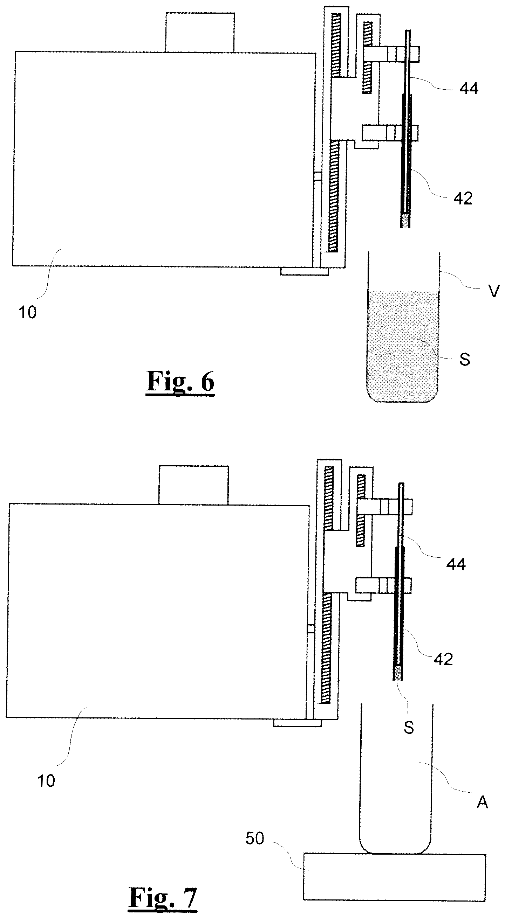

Daraufhin wird das Dosierwerkzeug 40 mittels der ersten Heb- und Senkeinrichtung 32 über den Rand des Vorratsbehälters V angehoben (Fig. 6). Eventuelle aus dem unteren Ende des Glasröhrchens 42 herausragende Substanzteile werden zuvor durch eine Seitwärtsbewegung des Dosierkopfs 30 abgestreift. Mit der ersten Waage 10 wird die Menge (Gewicht) der auf diese Weise vom Dosierwerkzeug 40 aufgenommenen Substanz S gemessen. Falls die aufgenommene Substanzmenge grösser ist als eine vorbestimmte nominelle Dosiermenge, wird unter der Kontrolle der ersten Waage 10 die überschüssige Substanzmenge aus dem Glasröhrchen 42 zurück in den Vorratsbehälter V ausgestossen. Dazu wird der Glasstempel 44 mittels der zweiten Heb-und Senkeinrichtung 34 relativ zum in der ersten Heb- und Senkeinrichtung 32 stationär gehaltenen Glasröhrchen 42 soweit abgesenkt, bis die erste Waage 10 eine bestimmte Substanzmenge detektiert. Dieses Trimmen der Dosiermenge durch Ausstossen der überschüssigen Substanzmenge ist in Fig. 9c dargestellt. Das Trimmen kann auch in mehreren Schritten iterativ erfolgen (Feedback-Loop).

Nun wird der Dosierkopf 30 mit dem in diesem gehalterten Dosierwerkzeug 40 mittels des Roboterarms 20 über einen auf der zweiten Waage 50 bereitgestellten Substanzaufnahmebehälter A gefahren, in welchen die Substanz S zudosiert werden soll (Fig. 7). Beim Substanzautnahmebehälter A handelt es sich vielfach um einen ganz kleinen Behälter (Vial), der zusammen mit vielen anderen Substanzaufnahmebehältern matrixförmig in einer Aufnahmeplatte (Rack) angeordnet ist.

Anschliessend wird mittels der zweiten Heb- und Senkeinrichtung 34 der Glasstempel 44 des Dosierwerkzeugs 40 bei durch das erste Greifwerkzeug 36 stationär gehaltenem Glasröhrchen 42 nach unten bewegt und dadurch die im Glasröhrchen 42 enthaltene Substanzmenge aus dem Glasröhrchen 42 in den Substanzaufnahmebehälter A ausgestossen (Fig. 8 und Fig. 9d). Mittels der zweiten (genaueren) Waage 50 kann die tatsächlich zudosierte Substanzmenge kontrolliert werden. Die tatsächliche Dosiermenge kann auch in geeigneter Weise protokolliert und dem abgefüllten Substanzaufnahmebehälter zugeordnet werden. In den meisten Fällen genügt es, wenn die Dosierung selbst nur einigermassen präzis ist, die tatsächliche Dosiermenge dafür aber höchst exakt bekannt ist.

Sofern ein weiterer Substanzaufnahmebehälter A mit derselben Substanz befüllt werden soll oder falls die auf diese Weise dosierte Substanzmenge die benötigte Substanzmenge noch nicht erreicht hat, wird der eben beschriebene Verfahrenszyklus gemäss den Figuren 5-8 wiederholt (Eintauchen, Trimmen, Ausstossen).

Falls eine andere Substanz abgefüllt werden soll, wird zunächst der Dosierkopf 30 über einem Abfallbehälter positioniert und das Dosierwerkzeug 40 dort entsorgt (verworfen). Dazu werden ganz einfach die Greifwerkzeuge 36 und 38 geöffnet, so dass das Dosierwerkzeug 40 nach unten in den Abfallbehälter herausfällt. Anschliessend wird wieder ein neues Dosierwerkzeug 40 aus dem Aufnahmegestell 46 aufgenommen (Fig. 4), und die Dosierung der nächsten Substanz erfolgt wieder gemäss dem vorstehend anhand der Fig. 5-8 beschriebenen Verfahrensablauf (Eintauchen, Trimmen, Ausstossen).

Die vorstehend beschriebenen Abläufe werden, wie schon erwähnt, von der Steuerelektronik 100 gesteuert. Die programmierbare Steuerelektronik ist dazu für die Durchführung der folgenden Abläufe ausgebildet:
- Bewegen des Dosierkopfs in vorzugsweise drei Raumrichtungen mittels des Roboterarms 20
- Einspannen eines (ausgewählten) Dosierwerkzeugs 40 in den Dosierkopf 30 durch Öffnen und Schliessen der Greifwerkzeuge 36 und 38
- Positionieren des Dosierwerkzeugs 40 über dem Substanzvorratsbehälter V
- Absenken des ganzen Dosierkopfs 30 oder alternativ nur des Dosierwerkzeugs 40 zum Eintauchen des Dosierwerkzeugs 40 in die im Substanzvorratsbehälter befindliche Substanz S und dadurch Aufnehmen von Substanz in das Dosierwerkzeug
- Anheben des Dosierwerkzeugs 40, vorzugsweise mittels der ersten Heb- und Senkeinrichtung 32
- Auslesen der ersten Waage 10 und ggf. zweiten Waage 50 und Auswertung der Messergebnisse der Waage(n)
- Absenken des Glasstempels 44 des Dosierwerkzeugs 40 relativ zum Glasröhrchen 42 mittels der zweiten Heb- und Senkeinrichtung 34 zur Ausstossung überschüssiger Substanz unter der Kontrolle der ersten Waage 10, gegebenenfalls iterativ
- Positionieren des Dosierwerkzeugs 40 über dem Substanzaufnahmebehälter A
- Absenken des Glasstempels 44 des Dosierwerkzeugs 40 relativ zum Glasröhrchen 42 mittels der Heb- und Senkeinrichtung 34 zur vollständigen Ausstossung von Substanz aus dem Dosierwerkzeug 40 in den Substanzaufnahmebehälter A
- Auswerfen des Dosierwerkzeugs 40 aus dem Dosierkopf 30 durch Öffnen der Greifwerkzeuge 36 und 38.

Im Aufnahmegestell 46 ist vorzugsweise eine grosse Zahl von Dosierwerkzeugen 40 vorrätig gehalten. Vorteilhafterweise sind Dosierwerkzeuge mit unterschiedlichen Dimensionen bzw. lichten Weiten (Innendurchmessern) von deren Glasröhrchen 42 vorhanden. Die Innendurchmesser können beispielsweise in einem Bereich von 0.1 mm bis etwa 5 mm liegen, vorzugsweise mit einer Abstufung von beispielsweise etwa 0.5 mm. Bevorzugt liegen die Innendurchmesser in einem Bereich von 0.1 bis 2 mm, speziell in einem Bereich von 0.1 bis 1 mm. Auf diese Weise können durch entsprechende Auswahl der Dosierwerkzeuge verschiedene Dosiermengen von einigen Hundert mg bis weit hinunter in den Sub-Milligramm-Bereich erreicht werden. Da der programmierbaren Steuerelektronik 100 durch entsprechende Eingaben ja bekannt ist, wie gross die geforderte Dosiermenge sein soll, kann sie passende Dosierwerkzeuge 40 gezielt selbsttätig auswählen. Ausserdem kann durch Verstellung des Glasstempels im Glasröhrchen des Dosierwerkzeugs das Fassungsvolumen der Dosierkammer angepasst werden.

Der Einsatz von Dosierwerkzeugen 40 mit unterschiedlichen Durchmessern der Glasröhrchen 42 hat aber noch einen weiteren Vorteil insofern, als sich dadurch sehr einfach eine rasche iterative Annäherung an die jeweils geforderte Soll-Dosiermenge realisieren lässt. Dabei werden zunächst eine oder mehrere Dosierdurchgänge mit dem grösstmöglichen Dosierwerkzeug durchgeführt. Unter grösstmöglichem Dosierwerkzeug wird dabei ein solches verstanden, dessen Glasröhrchen 42 für eine nominelle Dosiermenge ausgelegt ist, die möglichst nahe an die geforderte Soll-Dosiermenge herankommt, diese aber nicht übersteigt. Mittels der ersten Waage 10 und/oder der zweiten Waage 50 wird nach jedem Dosierdurchgang die noch erforderliche Restdosiermenge bestimmt. Wenn die Restmenge kleiner ist als die nominelle Dosiermenge des gerade eingesetzten Dosierwerkzeugs, wird dieses verworfen und ein neues, nächst kleineres Dosierwerkzeug eingesetzt, dessen nominelle Dosiermenge die Restdosiermenge nicht übersteigt. Damit werden nun so lange Dosierdurchgänge durchgeführt, bis die verbleibende Restdosiermenge wieder kleiner ist als die nominelle Dosiermenge des eingesetzten Dosierwerkzeugs. Dieser Vorgang wird mit zunehmend kleineren Dosierwerkzeugen, also Dosierwerkzeugen mit kleineren Durchmessern ihrer Glasröhrchen 42, so lange fortgesetzt, bis die geforderte Soll-Dosiermenge innerhalb einer vorgegebenen Toleranz erreicht ist. Anstelle gegebenenfalls ein kleineres Dosierwerkzeug zu wählen, kann auch das Fassungsvermögen der Dosierkammer des Dosierwerkzeugs entsprechend angepasst werden.

Für eine hohe Dosierpräzision ist es wichtig, dass die Glasstempel 44 möglichst passgenau (dicht) in den Glasröhrchen 42 der Dosierwerkzeuge 40 gleiten. Anzustreben ist eine Genauigkeit (Spiel) von etwa 0.01 mm. Die Wandstärken der Glasröhrchen 42 betragen abhängig von ihrem Durchmesser vorzugsweise typisch etwa 0.03 bis etwa 0.2 mm, bevorzugt etwa 0.03 bis etwa 0.1 mm. Die Längen der Glasröhrchen 42 betragen typisch etwa 70 mm, die Längen der Glasstempel 44 sind etwas grösser, typisch etwa 80 mm.

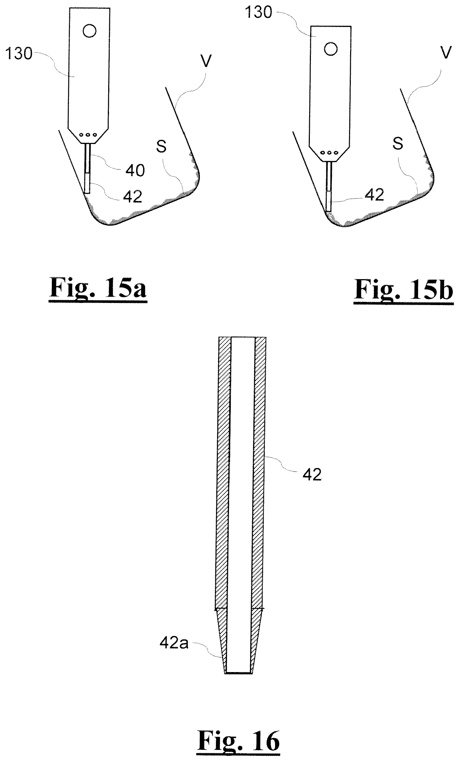

Die Gestalt des Röhrchens 42 verleiht diesem trotz Dünnwandigkeit eine relativ grosse Steifigkeit, welche für das Einstechen in feste(re) Substanzen oder kompaktere Pulver von Bedeutung ist. Die Dünnwandigkeit ist ebenfalls für das Einstechen wichtig. Zusätzlich oder alternativ kann, wie dies in Fig. 16 dargestellt ist, ein Ende 42a des Röhrchens 42 auch angeschärft bzw. schneidenartig ausgebildet sein. Weiters kann es auch vorteilhaft sein, das Eintauch- bzw. Einstechende des Röhrchens 42 sich nach aussen (geringfügig) erweiternd auszubilden.

Bei jedem Wechsel der Dosierwerkzeuge 40, sei es, weil eine andere Dimension des Glasröhrchens benötigt wird oder weil eine andere Substanz dosiert werden soll, wird das zuvor benutzte Dosierwerkzeug verworfen, also nicht mehr verwendet. Dadurch werden Kontaminationsprobleme mit grösstmöglicher Sicherheit vermieden. Die erfindungsgemässe Dosiervorrichtung ermöglicht dieses Konzept durch den Einsatz der speziell ausgebildeten, vollständig aus Glas bestehenden Dosierwerkzeuge 40, die als einzige Teile der gesamten Dosiervorrichtung mit den abzufüllenden Substanzen in Berührung kommen. Die Dosierwerkzeuge 40 bestehen nur aus zwei einfachen Glaskomponenten (Glasröhrchen 42 und Glasstempel 44), die einfach und kostengünstig als Massenartikel herstellbar sind, so dass ihr Einsatz als Wegwerfteile wirtschaftlich vertretbar ist.

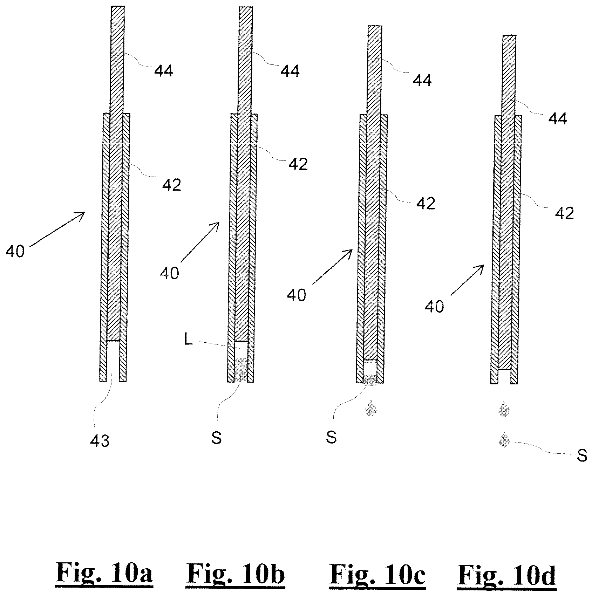

Die Figuren 10a-d zeigen eine Besonderheit beim Aufnehmen und Abgeben einer flüssigen Substanz in das bzw. aus dem Dosierwerkzeug 40. Fig. 10a entspricht Fig. 9a. Fig. 10b zeigt das Dosierwerkzeug 40 im in die aufzunehmende flüssige Substanz S eingetauchten Zustand. In der Dosierkammer 43 befindet sich zwischen dem Glasstempel 44 und dem Flüssigkeitspfropfen S ein Luftpolster L. Die Figuren 10c und 10d zeigen, wie die Substanz S durch stossweises Absenken des Glasstempels 44 tröpfchenweise aus der Dosierkammer 43 ausgestossen werden kann. Der Glasstempel wird dabei sehr schnell bewegt und erzeugt so Druckimpulse, die die flüssige Substanz tröpfchenweise ausstossen.

Beim vorstehend beschriebenen Verfahrensablauf dient die erste Waage als Entscheidungswaage (Wurde eine genügende Substanzmenge aufgenommen?) und die zweite Waage als eigentliche Messwaage (Welche Substanzmenge wurde effektiv zudosiert?).

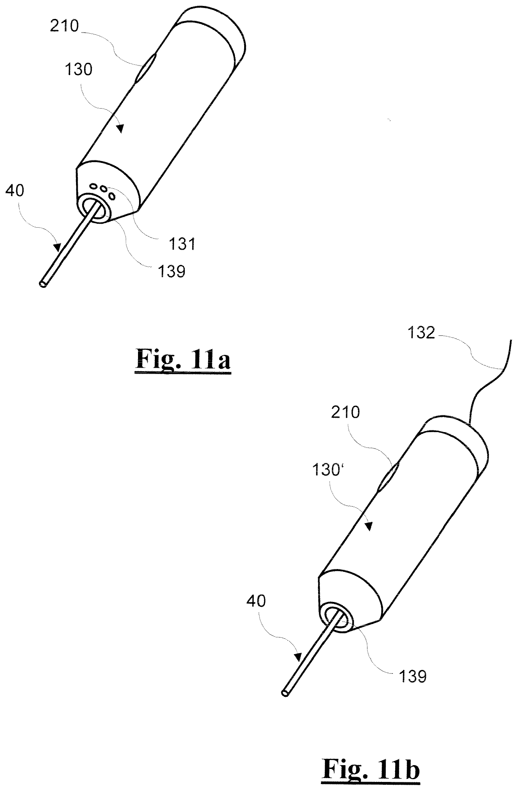

Im Folgenden wird anhand der Figuren 11a-b, 12a-c und 13a-i eine etwas einfachere Ausführungsform der erfindungsgemässen Dosiervorrichtung erläutert. Der grundlegendste Unterschied zur vorstehend beschriebenen Ausführungsform besteht darin, dass der Dosierkopf der Dosiervorrichtung als selbständiges und manuell zu bewegendes Element ausgebildet ist. Die folgende Beschreibung beschränkt sich daher im Wesentlichen auf die Ausbildung des Dosierkopfs sowie auf einen beispielsweisen Verfahrensablauf unter Einsatz eines solchen selbständigen Dosierkopfs.

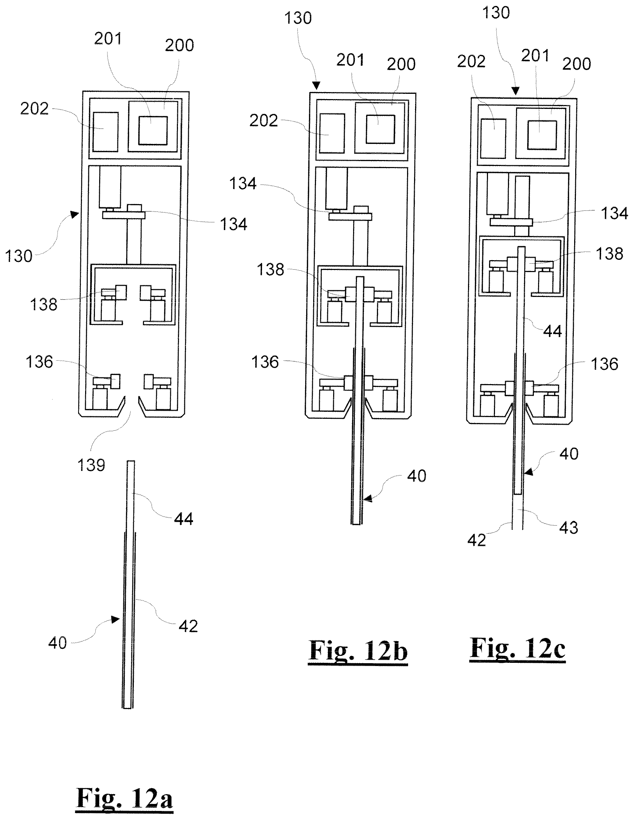

Die Figuren 11a und 11b zeigen zwei Varianten eines Dosierkopfs 130 bzw. 130'. In beiden Varianten hat der Dosierkopf beispielsweise eine im Wesentlichen zylindrische äussere Form. Bei der Variante der Fig. 11a sind am Dosierkopf 130 elektrische Kontakte 131 vorgesehen, über welche im Dosierkopf befindliche elektrische Komponenten mit externen Komponenten, z.B. einer Waage oder einer übergeordneten Steuerung oder einer Ladestromquelle, verbindbar sind. Bei der Variante der Fig. 11b erfolgt die elektrische Verbindung des Dosierkopfs 130' nach aussen über ein Kabel 132. Bei einer dritten, nicht dargestellten Variante könnte auch eine drahtlose Verbindung vorgesehen sein. Bei diesen Varianten sind am Dosierkopf 130 bzw. 130' aussen Bedienungsorgane 210 vorgesehen, die mit einer im Dosierkopf befindlichen internen Steuerung 200 (Fig. 12a) zusammenarbeiten. Die Bedienungsorgane 210 können z.B. in Form eines an sich bekannten Funktionsschaltknopfs mit z.B. mehreren Schaltfunktionen ausgebildet sein.

An seiner einen Stirnseite ist der Dosierkopf 130 bzw. 130' mit einer konisch ausgebildeten Führungsöffnung 139 versehen, durch welche ein Dosierwerkzeug 40 in den Dosierkopf eingesetzt werden kann. Näheres dazu ist nachfolgend erläutert.

Der innere Aufbau des Dosierkopfs 130 geht aus Fig. 12a hervor. Der Dosierkopf 130 umfasst ein erstes Greifwerkzeug 136, ein zweites Greifwerkzeug 138 und eine Heb-und Senkeinrichtung 134 für das zweite Greifwerkzeug 138. Ferner umfasst der Dosierkopf 130 die schon erwähnte interne Steuerung 200 sowie eine aufladbare Stromquelle bzw. einen Akkumulator 202. Beim Dosierkopf 130' kann die Stromversorgung über das Kabel 132 erfolgen, so dass der Akkumulator nicht unbedingt erforderlich ist.

Die beiden Greifwerkzeuge 136 und 138 und die Heb- und Senkeinrichtung 134 sind konstruktiv und funktionell im Prinzip gleich wie die entsprechenden Komponenten des ersten Ausführungsbeispiels der Dosiervorrichtung und bedürfen deshalb keiner weiteren Erläuterung. Die beiden Greifwerkzeuge 136 und 138 und die Heb- und Senkeinrichtung 134 werden von der internen Steuerung angesteuert, wobei das Öffnen und Schliessen der Greifwerkzeuge und das Heben und Senken des zweiten Greifwerkzeugs relativ zum ersten Greifwerkzeug manuell über die mit der internen Steuerung 200 verbundenen Bedienungsorgane 210 ausgelöst wird.

Die interne Steuerung 200 ist mit einer Schnittstelle 201 ausgestattet, die zur Kommunikation nach aussen dient. Die Schnittstelle 201 ist entweder an die elektrischen Kontakte 131 oder an das Kabel 132 angeschlossen. Alternativ kann die Schnittstelle auch als Drahtlosverbindung ausgeführt sein.

Die Fig. 12a zeigt den Dosierkopf 130 in einer Position oberhalb eines Dosierwerkzeugs 40, wobei die beiden Greifwerkzeuge 136 und 138 offen sind. In Fig. 12b ist der Dosierkopf 130 auf das Dosierwerkzeug 40 aufgesetzt bzw. letzteres ist in den Dosierkopf 130 eingeführt, wobei die beiden Greifwerkzeuge 136 und 138 geschlossen sind und das Dosierwerkzeug 40 festhalten. In Fig. 12c ist das zweite Greifwerkzeug 138 mittels der Heb- und Senkeinrichtung 134 etwas nach oben verschoben, wodurch der Glasstempel 44 des Dosierwerkzeugs 40 etwas aus dem Glasröhrchen 42 herausgezogen ist und so eine Dosierkammer 43 am unteren Ende des Dosierwerkzeugs 40 entstanden ist.

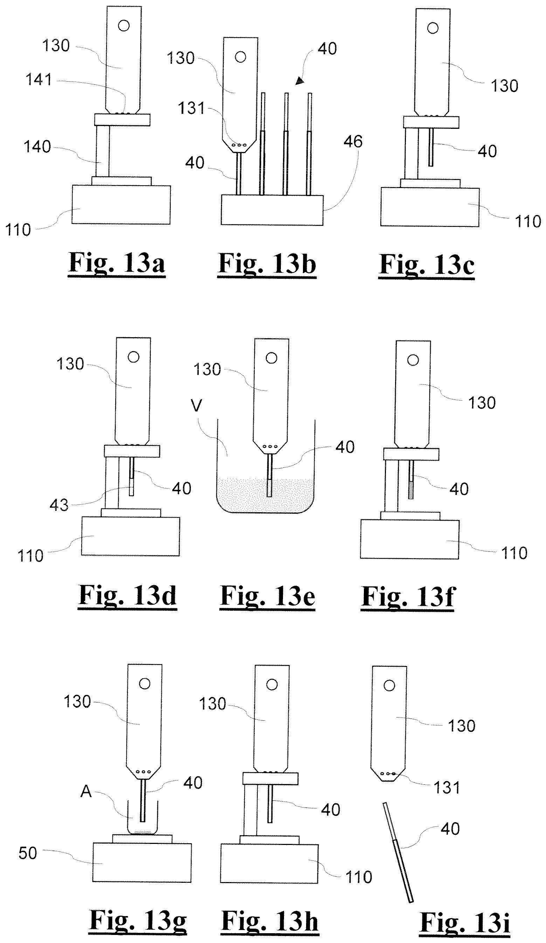

Die Figuren 13a-13i illustrieren die einzelnen Phasen eines typischen Dosiervorgangs unter Verwendung des Dosierkopfs 130.

Zu Beginn wird der Dosierkopf 130 mittels einer Waage 110 gewogen. Die Waage 110 ist mit einer Halterung 140 ausgestattet, in die der Dosierkopf 130 eingesetzt werden kann. Die Halterung 140 ist mit elektrischen Kontakten 141 ausgestattet, welche die elektrischen Kontakte 131 des Dosierkopfs 130 kontaktieren, wenn der Dosierkopf 130 in die Halterung 140 eingesetzt ist. Dabei kann z.B. der Akkumulator 202 im Dosierkopf 130 geladen werden oder (über den Funktionsschaltknopf 210) ein Wägevorgang ausgelöst werden.

Nach dem Wägevorgang wird der Dosierkopf 130 mit einem Dosierwerkzeug 40 bestückt. Dazu wird der Dosierkopf 130 manuell über ein Aufnahmegestell (Rack) 46 geführt und dann auf ein ausgewähltes Dosierwerkzeug 40 aufgesetzt (Fig. 13b). Mittels des Funktionsschaltknopfs 210 wird dann das Festklemmen des Dosierwerkzeugs 40 ausgelöst und der Dosierkopf 130 wieder in die Halterung 140 der Waage 110 eingesetzt und es wird wieder gewogen (Fig. 13c).

Daraufhin wird der Glasstempel 44 des Dosierwerkzeugs 40 etwas aus dessen Glasröhrchen 42 herausgezogen, so dass am unteren Ende des Dosierwerkzeugs 40 eine Aufnahmekammer 43 entsteht (Fig. 13d).

Im nächsten Schritt wird der Dosierkopf 130 manuell über ein Vorratsgefäss V mit aufzunehmender Substanz geführt und in diese eingetaucht, wodurch sich die Aufnahmekammer mit der aufzunehmenden Substanz füllt (Fig. 13e).

Daraufhin wird der Dosierkopf 130 wieder auf die Waage 110 gestellt und geprüft, ob die aufgenommene Substanzmenge ausreichend ist (Fig. 13f). Bei einer zu grossen Menge (mehr als dosiert werden soll) wird ein Teil wieder in den Vorratsbehälter V ausgestossen und nochmals gewogen.

Anschliessend wird der Dosierkopf 130 manuell über dem Substanzaufnahmebehälter (Zielbehälter) A positioniert und die in der Aufnahmekammer 43 des Dosierwerkzeugs 40 befindliche Substanzmenge in den Substanzaufnahmebehälter A ausgestossen. Der Substanzaufnahmebehälter A befindet sich dabei auf einer weiteren Waage 50, mit der die zudosierte Substanzmenge hochpräzis gemessen werden kann (Fig. 13g).

Daraufhin wird der Dosierkopf 130 wieder gewogen (Fig. 12h) und schliesslich das Dosierwerkzeug 40, falls es nicht wiederverwendet werden soll, ausgeworfen (Fig. 12i).

Der Dosiervorgang kann auch mit diesem Dosierkopf 130 wie schon weiter oben beschrieben in mehreren Durchgängen erfolgen.

Durch das Wägen des Dosierkopfs 130 (mit und ohne Substanz) in den verschiedenen Phasen des Dosierverfahrens lässt sich sehr genau bestimmen, wieviel Substanz tatsächlich dosiert wurde, und es lassen sich auch Rückschlüsse ziehen auf den aufzunehmenden Substanzüberschuss, der je nach Art der Substanz und den eingesetzten Dosierwerkzeugen unterschiedlich sein kann. So lässt sich das Dosierwerkzeug gewissermassen kalibrieren, wodurch sich der Arbeitsfluss für weitere Dosiervorgänge mit der jeweiligen Substanz erleichtern bzw. optimieren lässt. Analoges gilt auch für das Ausführungsbeispiel der Figuren 1-8 sowie das nachstehend beschriebene weitere Ausführungsbeispiel.

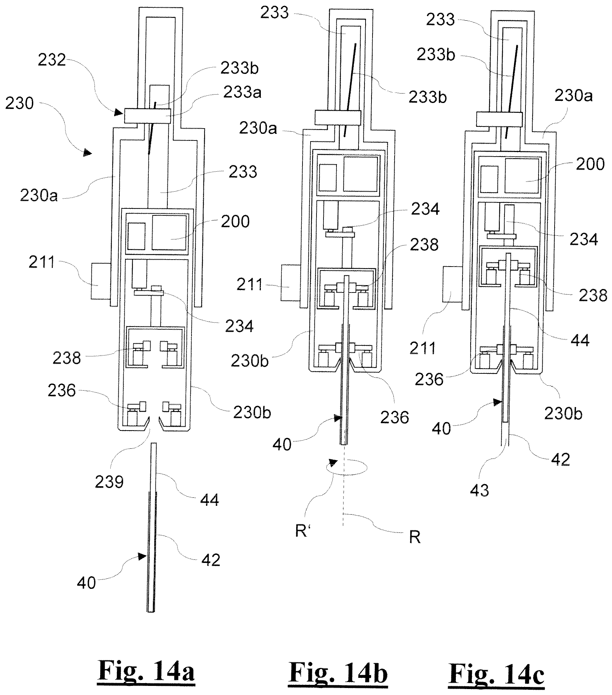

Im Folgenden wird anhand der Figuren 14a-e eine weitere Ausführungsform der erfindungsgemässen Dosiervorrichtung erläutert. Bei dieser Ausführungsform ist der als Ganzes mit 230 bezeichnete Dosierkopf wieder zur Befestigung an der ersten Waage 10 (Fig. 1) vorgesehen, sonst aber weitgehend ähnlich wie der Dosierkopf 130 der Ausführungsform gemäss den Figuren 11a und 12a-c ausgebildet. Die folgende Beschreibung beschränkt sich daher im Wesentlichen auf die Besonderheiten des Dosierkopfs 230 selbst sowie auf einen beispielsweisen Verfahrensablauf unter Einsatz dieses Dosierkopfs.

Der Dosierkopf 230 umfasst ein rohrförmiges Aussengehäuse 230a und ein in diesem axial verstellbares Innengehäuse 230b. Das Aussengehäuse 230a weist ein Verbindungsstück 211 auf, das zur mechanischen Befestigung am Waagbalken 11 an der ersten Waage 10 dient (Fig. 1). Das Verbindungsstück 211 kann auch (nicht dargestellte) elektrische Kontakte zur Verbindung des Dosierkopfs mit einer externen Steuerung aufweisen. Im Aussengehäuse 230a ist eine erste Heb- und Senkeinrichtung 232 angeordnet, welche eine mit dem Innengehäuse 230b verbundene Spindel 233 und einen Spindelmotor 233a umfasst. An der Spindel 233 ist eine schraubenlinienförmige Nut 233b vorgesehen. Mittels des Spindelmotors 233a kann das Innengehäuse 230b axial im Aussengehäuse 230a gehoben und gesenkt werden, wobei das Innengehäuse 230b gleichzeitig um seine Achse R in die eine bzw. die andere Richtung gedreht wird (Pfeile R' bzw. R").

Im Innengehäuse 230b sind wie beim Ausführungsbeispiel der Figuren 11a und 12a-c eine erstes und eine zweites Greifwerkzeug 236 bzw. 238 sowie eine zweite Heb- und Senkeinrichtung 234 angeordnet, wobei das zweite Greifwerkzeug 238 mittels der zweiten Heb- und Senkeinrichtung 234 relativ zum ortsfesten ersten Greifwerkzeug 236 angehoben bzw. abgesenkt werden kann. Am unteren Ende des Innengehäuses 230b ist eine konisch ausgebildeten Führungsöffnung 239 vorgesehen, durch welche ein Dosierwerkzeug 40 in den Dosierkopf 230 eingesetzt werden kann. Näheres dazu ist nachfolgend erläutert.

Die beiden Greifwerkzeuge 236 und 238 und die erste und zweite Heb- und Senkeinrichtung 232 und 234 sind konstruktiv und funktionell im Prinzip gleich wie die entsprechenden Komponenten des ersten Ausführungsbeispiels der Dosiervorrichtung und bedürfen deshalb keiner weiteren Erläuterung. Die beiden Greifwerkzeuge 236 und 238 und die beiden Heb- und Senkeinrichtung 232 und 234 werden wie beim ersten Ausführungsbeispiel von der externen Steuerung 100 (Fig. 1) angesteuert, wobei der Dosierkopf 230 über elektrische Kontakte oder drahtlos mit der Steuerung 100 in Verbindung steht. Selbstverständlich kann der Dosierkopf 230 auch mit einer internen Steuerung 200 ausgestattet sein, welche analoge Funktionen wie diejenige des Dosierkopfs 130 aufweisen kann.

Die Fig. 14a zeigt den Dosierkopf 230 in einer Ausgangsposition, noch ohne eingesetztes Dosierwerkzeug 40.

In Fig. 14b ist das Dosierwerkzeug 40 eingesetzt, wobei das erste und das zweite Greifwerkzeug 236 bzw. 238 das Glasröhrchen 42 bzw. den Glasstempel 44 des Dosierwerkzeugs 40 festhalten und das Innengehäuse 230b nach oben in das Aussengehäuse 230a des Dosierkopfs 230 eingezogen ist. Bei der Einzugsbewegung dreht sich das Innengehäuse 230b und damit das in diesem eingesetzte Dosierwerkzeug 40 um seine Längsachse R etwas in Richtung des Pfeils R'.

In Fig. 14c ist der Glasstempel 44 des Dosierwerkzeugs 40 etwa aus dem Glasröhrchen 42 herausgezogen, so dass eine Dosierkammer 43 entsteht.

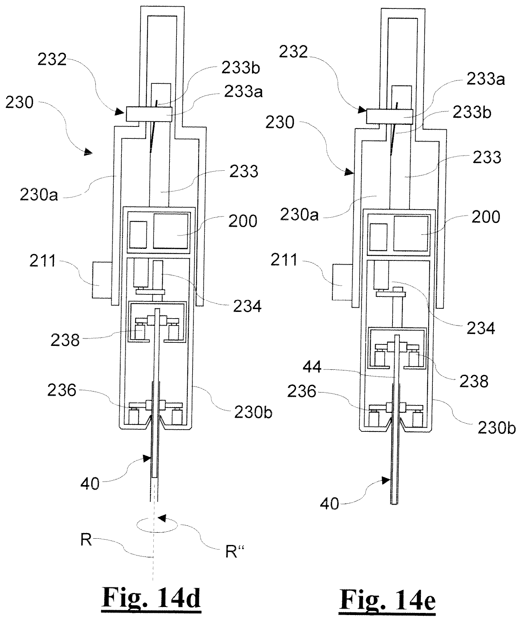

In dieser Konfiguration wird der Dosierkopf 230 im praktischen Einsatz über einem (nicht dargestellten) Vorratsgefäss positioniert und das Innengehäuse 230b abgesenkt, so dass das Dosierwerkzeug 40 in die im Vorratsgefäss enthaltene Substanz eintaucht bzw. einsticht, so dass eine bestimmte Menge der Substanz vom Dosierwerkzeug aufgenommen wird. Bei der Absenkbewegung des Innengehäuses 230b dreht sich dieses und damit das Dosierwerkzeug 40 um dessen Längsachse R in Richtung des Pfeils R". Die Drehbewegung des Dosierwerkzeugs 40 erleichtert das Einstechen im Falle von Substanzen mit relativ fester Konsistenz. Die Drehbewegung verhindert ausserdem ein Festsetzen des Dosierwerkzeugs in der Substanz und erlaubt bei gewissen Substanzen ein Herausbohren eines Substanzpfropfens. Die Fig. 14d zeigt den Dosierkopf 230 mit abgesenktem Innengehäuse 230b. Anschliessend wird die vom Dosierwerkzeug 40 aufgenommene Substanzmenge durch Absenken des Glasstempels 44 ganz oder teilweise aus dem Dosierwerkzeug 40 ausgestossen (Fig. 14e). Die Drehbewegung des Dosierwerkzeugs 40 kann übrigens sinngemäss auch beim Ausführungsbeispiel gemäss den Figuren 1-8 implementiert werden.

Beim praktischen Einsatz des Dosierkopfs 230 sind die einzelnen Schritte des Dosierverfahrens gleich wie bei dem anhand der Figuren 1-8 beschriebenen Ausführungsbeispiel der Dosiervorrichtung, mit dem einzigen Unterschied, dass sich bei diesem Dosierkopf das Dosierwerkzeug 40 beim Absenken zusätzlich noch etwas um seine Achse dreht. Eine weitere Erläuterung erübrigt sich daher.

In manchen Fällen liegt die zu dosierende Substanz im Vorratsbehälter V in kristallisierter Form vor, so wie dies in den Figuren 15a-b illustriert ist. Die Substanz S bildet dabei oft eine Kruste am Boden und an den Seitenwänden des Vorratsbehälters. Mit der erfindungsgemässen Dosiervorrichtung bzw. deren Dosierwerkzeug 40 lässt sich auch eine solche kristallisierte bzw. verkrustete Substanz S abkratzen und aufnehmen. Dabei ist es von Vorteil, wenn die Wandstärke des Glasröhrchens 42 des Dosierwerkzeugs 40 relativ gering ist, vorzugsweise etwa im Bereich von 0.03 mm bis 0.2 mm, bevorzugt 0.03 mm bis 0.1 mm. Zum Aufnehmen der Substanz S wird der Vorratsbehälter V vorzugsweise schief gehalten, so dass das Dosierwerkzeug 40 beim Absenken des Dosierkopfs an der Seitenwand des Vorratsbehälters V entlang gleitet und die Substanz abschabt. Dieser Vorgang kann auch mehrmals wiederholt werden, bis eine genügende Substanzmenge in die Dosierkammer aufgenommen ist bzw. sich in einem Randbereich des Vorratsbehälters V ansammelt, die dann von dort aufgenommen werden kann.

Die erfindungsgemässe Dosiervorrichtung ermöglicht mit einem einzigen Typ von Dosierwerkzeug eine präzise 1:1-Dosierung von unterschiedlichsten Substanzen von pulverförmig, flüssig, ölig, klebend, zähflüssig, wachsartig bis schokoladeartig, apfelartig, kristallisiert, verkrustet etc. Der bisher bei vielen Dosiervorrichtungen mühselige Wechsel von Dosierwerkzeugen je nach Konsistenz der zu dosierenden Substanz entfällt daher vollständig. Die erfindungsgemässe Dosiervorrichtung ist für praktisch alle gängigen Vorratsbehälter und praktisch alle gängigen Substanzaufnahmebehälter ohne irgendwelche Adapter geeignet. Mit der erfindungsgemässen Dosiervorrichtung können hochpräzise Dosierungen in einem weiten Bereich durchgeführt werden, z.B. von einigen Hundert mg bis hinunter in den Sub-Milligramm-Bereich. Die erreichte Präzision ist in der Grössenordnung 0.01 mg und in den meisten Fällen besser als bei manueller Dosierung. Die Dosiervorrichtung kann je nach Ausführungsform vollautomatisch arbeiten und ist wesentlich schneller als die in vielen Fällen noch geübte manuelle Dosierung.

## Patentansprüche

1. Vorrichtung zur Dosierung einer Substanz, mit einem Dosierkopf (30; 130; 130'; 230) und mit einem am Dosierkopf (30; 130: 130'; 230) lösbar befestigten Dosierwerkzeug (40) zur Aufnahme und Abgabe von Substanz, **dadurch gekennzeichnet, dass** das Dosierwerkzeug (40) als ein im Wesentlichen zylindrisches Röhrchen (42) mit einem darin im Wesentlichen dicht gleitend verstellbar angeordneten Stempel (44) ausgebildet ist, wobei der Stempel (44) länger ist als das Röhrchen (42) und am oberen Ende des Röhrchens (42) aus diesem herausragt, dass der Dosierkopf (30; 130; 130'; 230) mit einem öffen- und schliessbaren ersten Greifwerkzeug (36; 136; 236) zur lösbaren Einspannung des Röhrchens (42) und mit einem öffen- und schliessbaren zweiten Greifwerkzeug (38; 138: 238) zur lösbaren Einspannung des Stempels (44) versehen ist, und dass der Dosierkopf (30; 130; 130'; 230) mit einer Heb- und Senkeinrichtung (34; 134; 234) versehen ist, mittels welcher das zweite Greifwerkzeug (38; 138; 238) relativ zum ersten Greifwerkzeug (36; 136; 236) und dadurch der Stempel (44) im Röhrchen (42) heb- und senkbar ist.

2. Vorrichtung nach Anspruch 1, **dadurch gekennzeichnet, dass** das Röhrchen (42) und/oder der Stempel (44) als Glasröhrchen bzw. Glasstempel ausgebildet ist bzw. sind.

3. Vorrichtung nach Anspruch 1 oder 2, **dadurch gekennzeichnet, dass** der Dosierkopf (130; 130') eine interne Steuerung (200) für die Heb- und Senkeinrichtung (134) sowie das erste und das zweite Greifwerkzeug (136, 138) sowie mit der internen Steuerung (200) zusammenarbeitende Bedienungsorgane (210) aufweist.

4. Vorrichtung nach Anspruch 3, **dadurch gekennzeichnet, dass** die interne Steuerung (200) eine Schnittstelle (201) zur Kommunikation mit einer externen Steuerung und/oder zu einer externen Ladestromquelle aufweist.

5. Vorrichtung nach einem der Ansprüche 1-4, **dadurch gekennzeichnet, dass** sie eine erste Waage (110) für den Dosierkopf (130; 130') aufweist und dass die Waage (110) mit einer Halterung (140) für den Dosierkopf (130; 130') versehen ist.

6. Vorrichtung nach Anspruch 5, **dadurch gekennzeichnet, dass** die Halterung (140) mit elektrischen Kontakten (141) ausgestattet ist, welche zur Zusammenarbeit mit entsprechenden elektrischen Kontakten (131) am Dosierkopf (130) ausgebildet sind.

7. Vorrichtung nach Anspruch 1 oder 2, **dadurch gekennzeichnet, dass** sie eine erste Waage (10) umfasst und dass der Dosierkopf (30; 230) so an der Waage (10) angeordnet ist, dass die Waage (10) das Gewicht des gegebenenfalls aufgenommene Substanz enthaltenden Dosierkopfs (30; 230) misst.

8. Vorrichtung nach Anspruch 7, **dadurch gekennzeichnet, dass** der Dosierkopf (30; 230) mit einer zweiten Heb- und Senkeinrichtung (32; 232) ausgestattet ist, mittels welcher das erste Greifwerkzeug (36; 236) zusammen mit der erstgenannten Heb-und Senkeinrichtung (34; 234) für das zweite Greifwerkzeug (38; 238) heb- und senkbar ist.

9. Vorrichtung nach Anspruch 7 oder 8, **dadurch gekennzeichnet, dass** die Waage (10) an einem Roboterarm (20) befestigt ist, wobei der Dosierkopf (30; 230) mittels des Roboterarms (20) verstellbar ist, vorzugsweise in allen drei Raumrichtungen.

10. Vorrichtung nach einem der Ansprüche 7-9, **dadurch gekennzeichnet, dass** sie eine Steuerelektronik (100) für die Waage (10) und die Heb- und Senkeinrichtung (34; 234) sowie das erste und das zweite Greifwerkzeug (36, 38; 236, 238) aufweist.

11. Vorrichtung nach einem der vorangehenden Ansprüche, **dadurch gekennzeichnet, dass** sie zum Wägen eines Substanzaufnahmebehälters (A), der die abgegebene Substanz (S) aufnimmt, eine weitere Waage (50) aufweist.

12. Vorrichtung nach einem der vorangehenden Ansprüche, **dadurch gekennzeichnet, dass** das Röhrchen (42) einen Innendurchmesser im Bereich von 0.1 bis 5 mm, vorzugsweise 0.1 bis 2 mm, noch bevorzugter 0.1 bis 1 mm, aufweist.

13. Vorrichtung nach einem der vorangehenden Ansprüche, **dadurch gekennzeichnet, dass** das Röhrchen (42) eine Wandstärke im Bereich von 0.03 bis 0.2 mm, vorzugsweise 0.03 bis 0.1 mm, aufweist.

14. Vorrichtung nach einem der vorangehenden Ansprüche, **dadurch gekennzeichnet, dass** das Röhrchen (42) an einem Ende (42a) scharfkantig bzw. schneidenartig ausgebildet ist.

15. Vorrichtung nach Anspruch 8, **dadurch gekennzeichnet, dass** sie Drehmittel (233, 233a, 233b) aufweist, welche das Dosierwerkzeug (40) während seiner Anhebung und Absenkung durch die zweite Heb- und Senkeinrichtung (32; 232) um seine Längsachse (R) drehen.

16. Vorrichtung nach einem der vorangehenden Ansprüche, **dadurch gekennzeichnet, dass** sie ein Aufnahmegestell (46) für eine Anzahl von zumindest teilweise unterschiedlichen Dosierwerkzeugen (40) aufweist.

17. Dosierwerkzeug für den Einsatz in einer Vorrichtung zur Dosierung einer Substanz, **dadurch gekennzeichnet, dass** es als ein im Wesentlichen zylindrisches Röhrchen (42) mit einem darin im Wesentlichen dicht gleitend verstellbar angeordneten Stempel (44) ausgebildet ist, wobei der Stempel (44) länger ist als das Röhrchen (42) und am einen Ende des Röhrchens (42) aus diesem herausragt.

18. Dosierwerkzeug nach Anspruch 17, **dadurch gekennzeichnet, dass** das Röhrchen (42) und/oder der Stempel (44) als Glasröhrchen bzw. Glasstempel ausgebildet ist bzw. sind.

19. Dosierwerkzeug nach Anspruch 17 oder 18, **dadurch gekennzeichnet, dass** das Röhrchen (42) zylindrisch ist und einen Innendurchmesser im Bereich von 0.1 bis 5 mm, vorzugsweise 0.1 bis 2 mm, noch bevorzugter 0.1 bis 1 mm, aufweist.

20. Dosierwerkzeug nach einem der Ansprüche 17-19, **dadurch gekennzeichnet, dass** das Röhrchen (42) eine Wandstärke im Bereich von 0.03 bis 0.2 mm, vorzugsweise 0.03 bis 0.1 mm, aufweist.

21. Dosierwerkzeug nach einem der Ansprüche 17-20, **dadurch gekennzeichnet, dass** das Röhrchen (42) an einem Ende (42a) scharfkantig bzw. schneidenartig ausgebildet ist.

22. Verfahren zum Dosieren einer Substanz unter Verwendung einer Vorrichtung nach einem der Ansprüche 1-16, **gekennzeichnet durch** die folgenden Schritte:
- Einspannen des Dosierwerkzeugs (40) in den Dosierkopf (30; 130; 130'; 230) durch Öffnen und Schliessen der Greifwerkzeuge (36, 38; 136, 138; 236, 238)
- Positionieren des Dosierwerkzeugs (40) über einem Substanzvorratsbehälter (V)
- Absenken des Dosierwerkzeugs (40) zum Eintauchen bzw. Einstechen des Dosierwerkzeugs (40) in eine im Substanzvorratsbehälter befindliche Substanz (S) und dadurch Aufnehmen von Substanz in eine Substanzkammer (43) des Dosierwerkzeugs (40), vorzugsweise unter der Kontrolle einer Waage (10; 110)
- Anheben des Dosierwerkzeugs (40)
- gegebenenfalls Absenken des Stempels (44) des Dosierwerkzeugs (40) relativ zum Röhrchen (42) zur Ausstossung überschüssiger Substanz, vorzugsweise unter der Kontrolle einer Waage
- Positionieren des Dosierwerkzeugs (40) über einem Substanzaufnahmebehälter (A), und
- Absenken des Stempels (44) des Dosierwerkzeugs (40) relativ zum Röhrchen (42) zur vollständigen Ausstossung von Substanz aus dem Dosierwerkzeug (40) in den Substanzaufnahmebehälter (A).

23. Verfahren nach Anspruch 22, **dadurch gekennzeichnet, dass** die Dosierung in einzelnen Teildosierungen durchgeführt wird und dabei, vorzugsweise unter der Kontrolle einer Waage (10; 110), eine schrittweise Annäherung an eine geforderte Soll-Dosiermenge erfolgt.

24. Verfahren nach Anspruch 22 oder 23, **dadurch gekennzeichnet, dass** die vom Dosierwerkzeug (40) aufgenommene Substanzmenge unter der Kontrolle einer Waage (10; 110) getrimmt wird.

25. Verfahren nach einem der Ansprüche 22-24, **dadurch gekennzeichnet, dass** die tatsächliche Menge der in den Substanzaufnahmebehälter (A) dosierten Substanz mittels einer Waage (50) gemessen wird.

26. Verfahren nach einem der Ansprüche 22-25, **dadurch gekennzeichnet, dass** das Dosierwerkzeug (40) während des Eintauchens bzw. Einstechens in eine im Substanzvorratsbehälter befindliche Substanz (S) und vorzugsweise auch während des Herausziehens aus der Substanz (S) um seine Längsachse (R) gedreht wird.

27. Verfahren nach einem der Ansprüche 22-26, **dadurch gekennzeichnet, dass** in einem Aufnahmegestell (46) eine Anzahl von zumindest teilweise unterschiedlichen Dosierwerkzeugen (40) bereitgestellt wird und dass das einzuspannende Dosierwerkzeug aus diesen bereitgestellten Dosierwerkzeugen ausgewählt wird.

## Claims

1. Apparatus for metering a substance, having a metering head (30; 130; 130'; 230) and having a metering tool (40) releasably attached to the metering head (30; 130; 130'; 230) for taking up and dispensing substance, **characterized in that** the metering tool (40) is configured as an essentially cylindrical tubule (42) having a punch (44) disposed in it to slide adjustably, essentially forming a seal, wherein the punch (44) is longer than the tubule (42) and projects out of the tubule (42) at its upper end, that the metering head (30; 130; 130'; 230) is provided with a first gripping tool (36; 136; 236) that can open and close for releasable clamping of the tubule (42) and with a second gripping tool (38; 138; 238) that can open and close for releasable clamping of the punch (44), and that the metering head (30; 130; 130'; 230) is provided with a raising and lowering device (34; 134; 234), by means of which the second gripping tool (38; 138; 238) can be raised and lowered relative to the first gripping tool (36; 136; 236), and thereby the punch (44) can be raised and lowered in the tubule (42).

2. Apparatus according to claim 1, **characterized in that** the tubule (42) and/or the punch (44) is/are configured as a glass tubule and a glass punch, respectively.

3. Apparatus according to claim 1 or 2, **characterized in that** the metering head (130; 130') has an internal controller (200) for the raising and lowering device (134) as well as the first and the second gripping tool (136, 138), as well as operating elements (21) that work together with the internal controller (200).

4. Apparatus according to claim 3, **characterized in that** the internal controller (200) has an interface (201) for communication with an external controller and/or for an external charging current source.

5. Apparatus according to one of claims 1-4, **characterized in that** it has a first scale (110) for the metering head (130; 130') and that the scale (110) is provided with a holder (140) for the metering head (130; 130').

6. Apparatus according to claim 5, **characterized in that** the holder (140) is equipped with electrical contacts (141), which are configured for working together with corresponding electrical contacts (131) on the metering head (130).

7. Apparatus according to claim 1 or 2, **characterized in that** it comprises a first scale (10) and that the metering head (30; 230) is disposed on the scale (10) in such a manner that the scale (10) measures the weight of the metering head (30; 230), which might contain substance that has been taken up.

8. Apparatus according to claim 7, **characterized in that** the metering head (30; 230) is equipped with a second raising and lowering device (32; 232), by means of which the first gripping tool (36; 236) can be raised and lowered, together with the raising and lowering device (34; 234) for the second gripping tool (38; 238) first mentioned.

9. Apparatus according to claim 7 or 8, **characterized in that** the scale (10) is attached to a robot arm (20), wherein the metering head (30; 230) is adjustable by means of the robot arm (20), preferably in all three spatial directions.

10. Apparatus according to one of claims 7-9, **characterized in that** it has control electronics (100) for the scale (10) and the raising and lowering device (34; 234), as well as the first and the second gripping tool (36, 38; 236, 238).

11. Apparatus according to one of the preceding claims, **characterized in that** it has a further scale (50) for weighing a substance receptacle (A), which takes up the dispensed substance (S).

12. Apparatus according to one of the preceding claims, **characterized in that** the tubule (42) has an inside diameter in the range of 0.1 to 5 mm, preferably 0.1 to 2 mm, even more preferably 0.1 to 1 mm.

13. Apparatus according to one of the preceding claims, **characterized in that** the tubule (42) has a wall thickness in the range of 0.03 to 0.2 mm, preferably 0.03 to 0.1 mm.

14. Apparatus according to one of the preceding claims, **characterized in that** the tubule (42) is configured to have a sharp edge or configured like a blade at one end (42a).

15. Apparatus according to claim 8, **characterized in that** it has rotation means (233, 233a, 233b), which rotate the metering tool (40) about its longitudinal axis (R) while it is being raised and lowered by the second raising and lowering device (32; 232).

16. Apparatus according to one of the preceding claims, **characterized in that** it has a rack (46) for a number of at least partially different metering tools (40).

17. Metering tool for use in an apparatus for metering a substance, **characterized in that** it is configured as an essentially cylindrical tubule (42) having a punch (44) disposed in it to slide adjustably, essentially forming a seal, wherein the punch (44) is longer than the tubule (42) and projects out of the tubule (42) at one of its ends.

18. Metering tool according to claim 17, **characterized in that** the tubule (42) and/or the punch (44) is/are configured as a glass tubule or as a glass punch.

19. Metering tool according to claim 17 or 18, **characterized in that** the tubule (42) is cylindrical and has an inside diameter in the range of 0.1 to 5 mm, preferably 0.1 to 2 mm, even more preferably 0.1 to 1 mm.

20. Metering tool according to one of claims 17-19, **characterized in that** the tubule (42) has a wall thickness in the range of 0.03 to 0.2 mm, preferably 0.03 to 0.1 mm.

21. Metering tool according to one of claims 17-20, **characterized in that** the tubule (42) is configured with a sharp edge or configured like a blade at one end (42a).

22. Method for metering a substance, using an apparatus according to one of claims 1-16, **characterized by** the following steps:
- clamping the metering tool (40) into the metering head (30; 130; 130'; 230) by means of opening and closing the gripping tools (36, 38; 136, 138; 236, 238),
- positioning the metering tool (40) above a substance storage container (V),
- lowering the metering tool (40) to immerse or insert the metering tool (40) into a substance (S) situated in the substance storage container, and thereby taking substance up into a substance chamber (43) of the metering tool (40), preferably with monitoring by a scale (10; 110),
- raising the metering tool (40),
- if applicable, lowering the punch (44) of the metering tool (40) relative to the tubule (42) to eject excess substance, preferably with monitoring by a scale,
- positioning the metering tool (40) above a substance receptacle (A), and
- lowering the punch (44) of the metering tool (40) relative to the tubule (42) to completely eject substance out of the metering tool (40) into the substance receptacle (A).

23. Method according to claim 22, **characterized in that** the metering is carried out in individual partial metering steps, and during this process, preferably with monitoring by a scale (10; 110), step-by-step approximation to a required target metering amount takes place.

24. Method according to claim 22 or 23, **characterized in that** the amount of substance taken up by the metering tool (40) is trimmed with monitoring by a scale (10; 110).

25. Method according to one of claims 22-24, **characterized in that** the actual amount of the substance metered into the substance receptacle (A) is measured by means of a scale (50).

26. Method according to one of claims 22-25, **characterized in that** the metering tool (40) is rotated about its longitudinal axis (R) while it is immersed or inserted into a substance (S) contained in the substance storage container, and preferably also while it is pulled out of the substance (S).

27. Method according to one of claims 22-26, **characterized in that** a number of at least partially different metering tools (40) is made available in a rack (46), and that the metering tool to be clamped is selected from among these metering tools that have been made available.

## Revendications

1. Dispositif de dosage d'une substance, comportant une tête de dosage (30 ; 130 ; 130' ; 230) et un outil de dosage (40) fixé de façon amovible à la tête de dosage (30 ; 130 ; 130' ; 230) et destiné à recevoir et à distribuer une substance, **caractérisé en ce que**
l'outil de dosage (40) est réalisé sous la forme d'un tube (42) sensiblement cylindrique avec un poinçon (44) agencé dans celui-ci en étant mobile en glissement de façon sensiblement étanche, dans lequel le poinçon (44) est plus long que le tube (42) et dépasse hors du tube (42) à son extrémité supérieure,
la tête de dosage (30 ; 130 ; 130' ; 230) est pourvue d'un premier outil de préhension (36 ; 136 ; 236) susceptible d'être ouvert et fermé et destiné au serrage amovible du tube (42) et est pourvue d'un second outil de préhension (38 ; 138 ; 238) susceptible d'être ouvert et fermé et destiné au serrage amovible du poinçon (44), et
la tête de dosage (30 ; 130 ; 130' ; 230) est pourvue d'un moyen de levage et d'abaissement (34 ; 134 ; 234) permettant de lever et d'abaisser le second outil de préhension (38 ; 138 ; 238) par rapport au premier outil de préhension (36 ; 136 ; 236) et ainsi de lever et d'abaisser le poinçon (44) dans le tube (42).

2. Dispositif selon la revendication 1, **caractérisé en ce que** le tube (42) et/ou le poinçon (44) est/sont réalisé(s) sous forme de tube en verre ou de poinçon en verre.

3. Dispositif selon la revendication 1 ou 2, **caractérisé en ce que** la tête de dosage (130 ; 130') comprend une commande interne (200) pour le moyen de levage et d'abaissement (134) ainsi que le premier et le second outil de préhension (136, 138) et des organes de manipulation (210) coopérant avec la commande interne (200).

4. Dispositif selon la revendication 3, **caractérisé en ce que** la commande interne (200) présente une interface (201) pour la communication avec une commande externe et/ou vers une source de courant de charge externe.

5. Dispositif selon l'une des revendications 1 à 4, **caractérisé en ce qu'**il comprend une première balance (110) pour la tête de dosage (130 ; 130'), et **en ce que** la balance (110) est pourvue d'une monture (140) pour la tête de dosage (130 ; 130').

6. Dispositif selon la revendication 5, **caractérisé en ce que** la monture (140) est équipée de contacts électriques (141) qui sont réalisés pour coopérer avec des contacts électriques correspondants (131) sur la tête de dosage (130).

7. Dispositif selon la revendication 1 ou 2, **caractérisé en ce qu'**il comprend une première balance (10), et **en ce que** la tête de dosage (30 ; 230) est agencée sur la balance (10) de telle sorte que la balance (10) mesure le poids de la tête de dosage (30 ; 230) contenant le cas échéant une substance reçue.

8. Dispositif selon la revendication 7, **caractérisé en ce que** la tête de dosage (30 ; 230) est équipée d'un second moyen de levage et d'abaissement (32 ; 232) permettant de lever et d'abaisser le premier outil de préhension (36 ; 236) conjointement avec le premier moyen de levage et d'abaissement (34 ; 234) pour le second outil de préhension (38 ; 238).

9. Dispositif selon la revendication 7 ou 8, **caractérisé en ce que** la balance (10) est fixée à un bras robotique (20), la tête de dosage (30 ; 230) étant mobile au moyen du bras robotique (20), de préférence dans toutes les trois directions spatiales.

10. Dispositif selon l'une des revendications 7 à 9, **caractérisé en ce qu'**il comprend une électronique de commande (100) pour la balance (10) et pour le moyen de levage et d'abaissement (34 ; 234) ainsi que pour la premier et le second outil de préhension (36, 38 ; 236, 238).

11. Dispositif selon l'une des revendications précédentes, **caractérisé en ce qu'**il comprend une autre balance (50) pour peser un récipient de réception de substance (A) qui reçoit la substance (S) distribuée.

12. Dispositif selon l'une des revendications précédentes, **caractérisé en ce que** le tube (42) présente un diamètre intérieur dans la plage de 0,1 à 5 mm, de préférence de 0,1 à 2 mm, de préférence particulière de 0,1 à 1 mm.

13. Dispositif selon l'une des revendications précédentes, **caractérisé en ce que** le tube (42) présente une épaisseur de paroi dans la plage de 0,03 à 0,2 mm, de préférence de 0,03 à 0,1 mm.

14. Dispositif selon l'une des revendications précédentes, **caractérisé en ce qu'**à une extrémité (42a), le tube (42) est réalisé avec une arête vive ou à la manière d'un tranchant.

15. Dispositif selon la revendication 8, **caractérisé en ce qu'**il comprend des moyens de rotation (233, 233a, 233b) qui font tourner l'outil de dosage (40) autour de son axe longitudinal (R) pendant son levage et son abaissement par le second moyen de levage et d'abaissement (32 ; 232).

16. Dispositif selon l'une des revendications précédentes, **caractérisé en ce qu'**il comprend un bâti de réception (46) pour un certain nombre d'outils de dosage (40) au moins partiellement différents.

17. Outil de dosage pour l'utilisation dans un dispositif de dosage d'une substance, **caractérisé en ce qu'**il est réalisé sous la forme d'un tube (42) sensiblement cylindrique avec un poinçon (44) agencé dans celui-ci en étant mobile en glissement de façon sensiblement étanche, le poinçon (44) étant plus long que le tube (42) et dépassant hors du tube (42) à une extrémité du tube (42).

18. Outil de dosage selon la revendication 17, **caractérisé en ce que** le tube (42) et/ou le poinçon (44) est/sont réalisé(s) sous forme de tube en verre ou de poinçon en verre.

19. Outil de dosage selon la revendication 17 ou 18, **caractérisé en ce que** le tube (42) est cylindrique et présente un diamètre intérieur dans la plage de 0,1 à 5 mm, de préférence de 0,1 à 2 mm, de préférence particulière de 0,1 à 1 mm.

20. Outil de dosage selon l'une des revendications 17 à 19, **caractérisé en ce que** le tube (42) présente une épaisseur de paroi dans la plage de 0,03 à 0,2 mm, de préférence de 0,03 à 0,1 mm.

21. Outil de dosage selon l'une des revendications 17 à 20, **caractérisé en ce qu'**à une extrémité (42a), le tube (42) est réalisé avec une arête vive ou à la manière d'un tranchant.

22. Procédé de dosage d'une substance en utilisant un dispositif selon l'une des revendications 1 à 16, **caractérisé par** les étapes suivantes consistant à :
- serrer l'outil de dosage (40) dans la tête de dosage (30 ; 130 ; 130' ; 230) par ouverture et fermeture des outils de préhension (36, 38 ; 136, 138 ; 236, 238),
- positionner l'outil de dosage (40) au-dessus d'un réservoir de substance (V),
- abaisser l'outil de dosage (40) pour faire plonger ou pénétrer l'outil de dosage (40) dans une substance (S) située dans le réservoir de substance et ainsi recevoir de la substance dans une chambre à substance (43) de l'outil de dosage (40), de préférence sous le contrôle d'une balance (10 ; 110),
- lever l'outil de dosage (40),
- le cas échéant abaisser le poinçon (44) de l'outil de dosage (40) par rapport au tube (42) pour expulser un excédent de substance, de préférence sous le contrôle d'une balance,
- positionner l'outil de dosage (40) au-dessus d'un récipient de réception de substance (A), et
- abaisser le poinçon (44) de l'outil de dosage (40) par rapport au tube (42) pour expulser complètement la substance hors de l'outil de dosage (40) jusque dans le récipient de réception de substance (A).

23. Procédé selon la revendication 22, **caractérisé en ce que** le dosage est effectué en dosages partiels individuels en s'approchant par pas d'une quantité de dosage de consigne requise, de préférence sous le contrôle d'une balance (10 ; 110).

24. Procédé selon la revendication 22 ou 23, **caractérisé en ce que** la quantité de substance reçue par l'outil de dosage (40) est réglée sous le contrôle d'une balance (10 ; 110).

25. Procédé selon l'une des revendications 22 à 24, **caractérisé en ce que** la quantité réelle de la substance dosée dans le récipient de réception de substance (A) est mesurée au moyen d'une balance (50).

26. Procédé selon l'une des revendications 22 à 25, **caractérisé en ce que** l'outil de dosage (40) est tourné autour de son axe longitudinal (R) pendant la plongée ou la pénétration dans une substance (S) située dans le réservoir de substance et de préférence également pendant l'extraction hors de la substance (S).

27. Procédé selon l'une des revendications 22 à 26, **caractérisé en ce qu'**un certain nombre d'outils de dosage (40) au moins partiellement différents sont mis à disposition dans un bâti de réception (46), et **en ce que** l'outil de dosage à serrer est choisi parmi ces outils de dosage mis à disposition.
